# EUROPEAN PATENT APPLICATION

(11) **EP 2 659 933 A1**
(43) Date of publication of application: **06.11.2013**
(21) Application number: 13158596.0
(22) Date of filing: 21.01.2009
(51) Int. Cl.: A61P 9/00, A61K 31/155, A61K 31/7048, A61K 31/40, A61K 31/44, A61K 45/06, A61K 31/4422, A61K 31/4436, A61K 31/4439

(54) **Pharmaceutical combinations comprising specified age breaker and further drugs, i.a. antihypertensive drugs, antidiabetic drugs etc.**

(30) Priority: 25.01.2008 IN KA01522008
(62) Divisional of application: 09704262.6
(71) Applicant: Torrent Pharmaceuticals Ltd., Ahmedabad 380 009 (IN)
(72) Inventor: Dutt, Chaitanya, P.O. Bhat Dist. Gandhinagar 382 428 Gujarat (IN); Joshi, Deepa, P.O. Bhat Dist. Gandhinagar 382 428 Gujarat (IN); Gupta, Ram, P.O. Bhat Dist. Gandhinagar 382 428 Gujarat (IN); Chandra, Kumarprafull, P.O. Bhat Dist. Gandhinagar 382 428 Gujarat (IN)
(74) Representative: Klunker . Schmitt-Nilson . Hirsch

(57) **Abstract**

The present invention relates to a combination, such as a combined preparation or pharmaceutical composition comprising: (a) compound of formula (I), and/or (II) or a pharmaceutically acceptable salt thereof; (b) at least one therapeutic agent selected from the group consisting of: an antihypertensive agent; an antidiabetics agent; a hypolipidemic agent; an antiplatelet agent; an antiobesity agent; an antithrombotic agent; an agent for diabetic vascular complications; and an agent for treatment of heart failure; or a pharmaceutically acceptable salts thereof, optionally in presence of a pharmaceutically acceptable carrier for separate, simultaneous or sequential use. The present invention also relates to a use of such combination for the treatment of mammal including human being.

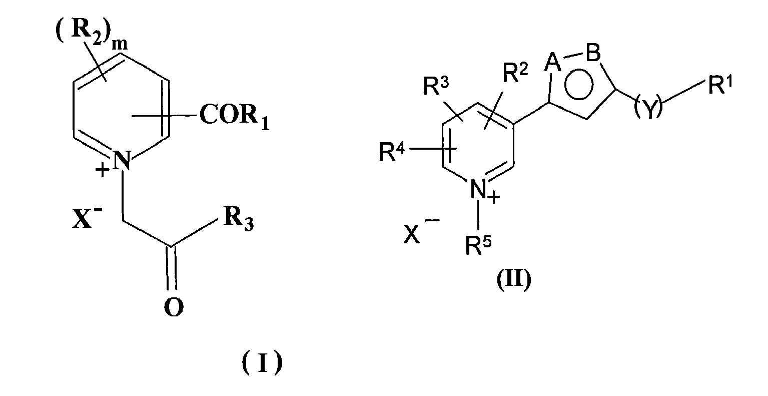

R₁, R₂, R₃, R¹, R², R³, R⁴, R⁵, X, Y, A and B and m are as defined in the specification.

## Description

### Field of the Invention:

The present invention relates to a combination, such as a combined preparation or pharmaceutical composition comprising: (a) compound of formula (I), and/or (II) as defined hereinafter or a pharmaceutically acceptable salt thereof; (b) at least one therapeutic agent selected from the group consisting of: an antihypertensive agent; an antidiabetics agent; a hypolipidemic agent; an antiplatelet agent; an antiobesity agent; an antithrombotic agent; an agent for diabetic vascular complications; and an agent for treatment of heart failure; or a pharmaceutically acceptable salts thereof, optionally in presence of a pharmaceutically acceptable carrier for separate, simultaneous or sequential use. The present invention also relates to a use of such combination for the manufacture of medicament for the treatment of mammal including human being.

### Background of the Invention:

There is an increasing body of evidence implicating Advanced Glycation Endproducts (AGEs) as one of the major cause of diabetic complications. AGEs are formed by a complex chain of reactions between reducing sugar such as glucose with proteins, resulting in the formation of multimeric complexes that trigger several pathological events (Aronson D & Rayfield EJ. Cardiovasc Diabetol 2002; 1: 1-10).

Due to the clinical significance of AGE formation, several successful therapeutic approaches have been tried based upon intervening in the accumulation of AGEs in vivo. One of the approach is to inhibit the formation of AGEs from its precursors, by the administration of therapeutic agents. In another approach for controlling levels of AGEs in tissues, therapeutic agent is administered which can reverse or break AGE cross-links, especially in those tissues in which AGE cross-links have already accumulated to levels which are responsible for subclinical or clinical pathology.

WO/01/25208 and WO/02/85897 discloses various novel compounds useful as AGE inhibitors and AGE breakers.

Diabetes is an important adult disease all over the world and it refers to a disease process caused due to multiple factors and characterized by elevated levels of plasma glucose or hyperglycemia. Worldwide, more than 171 million people have diabetes, and its prevalence is expected to double by 2030. (Padwal R et al, Diabetes Care, 2005; 28:736-44.) Persistent or uncontrolled hyperglycemia is associated with increased and premature morbidity and mortality. Often abnormal glucose homeostasis is associated both directly or indirectly with alterations of the lipid, lipoprotein and apolipoprotein metabolism and other metabolic and hemodynamic disease. Therefore patients with Type 2 diabetes mellitus are at especially increased risk of macrovascular and microvascular complications, including coronary heart disease, stroke, peripheral vascular disease, hypertension, nephropathy, neuropathy, and retinopathy. Therefore, therapeutical control of glucose homeostasis, lipid metabolism and hypertension are critically important in the clinical management and treatment of diabetes mellitus and prevention of comorbidity.

Diabetic complications, a major cause of death due to diabetes, occur by the damage of almost all organs in the body at 10-20 years after the onset of diabetes. These diabetic complications can progress even when diabetes is well controlled so as to recover the normal blood glucose concentration. Diabetes and Hypertension frequently coexist, leading to additive increase in the risk of life threatening cardiovascular events. It is well known that hypertension accelerates the course of micro and macrovascular complications of diabetes and hypertension often precedes type-II diabetes and vice versa.(Schutta MH, J Cardiometab. Syndr., 2007; 2(2): 124-30) Hypertension affects 40-60% of type 2 diabetics between the ages of 40-75. Hypertension is often unrecognized, resistant to treatment and undertreated, in spite of the fact that if hypertension is controlled this reduces diabetes related deaths, stroke, macrovascular disease, microvascular disease and heart failure.

People with diabetes are at increased risk of developing heart failure with the relative risk increasing by 10-15% per unit increase in glycated haemoglobin. Conversely, heart failure is present in 25-40% of all adults with diabetes. Moreover, people with heart failure have worse outcomes if they also have diabetes and it has been suggested that any level of hyperglycaemia is associated with increased rates of hospital admission, even in patients without manifest diabetes. (Eurich DT et al., BMJ, 2007; 335: 497 (8 September),) Diabetes mellitus is an independent risk factor for CHF and vice versa. Apart from CHD and hypertension, hyperglycaemia and insulin resistance are directly linked to the development of diastolic dysfunction and to CHF. (Fijchtenbusch M. et al., MMW Fortschr Med., 2007; 149(37): 41-4)

Patients with signs and symptoms of heart failure and a preserved left ventricular (LV) systolic function may have significant abnormalities in diastolic function. This condition is called diastolic heart failure (DHF) and is observed in about 40% of patients with chronic heart failure (CHF). Diabetes mellitus is one of the major risk factors for DHF. Diastolic dysfunction is observed in about 40% of patients with diabetes mellitus and correlates with poor glycemic control. Poor glycemic control is associated with a high incidence of heart failure in diabetic patients (Tsujino T et al., Am J Cardiovasc Drugs. 2006;6(4):219-30)

It has been observed that the usual treatments in heart failure have similar or lower efficacy in the diabetic patient, and treatment intolerance is frequent. Treatments used for diabetes often are poorly tolerated in case of heart failure (metformin, glitazones) (Cohen-Solal A & Logeart D.,Arch Mal Coeur Vaiss. 2007;100(6-7): 535-46)

Health problems such as heart disease, stroke, kidney disease, eye damage and foot problems that can lead to amputations are far more prevalent in people with type 2 diabetes than in the general population. In USA, an estimated three out of five people with diabetes (57.9 percent) have one or more of the complications associated with diabetes. Cardiovascular disease is responsible for between 50% and 80% of deaths in people with diabetes. Studies suggest that up to 50% of people with diabetes are affected to some degree of diabetic neuropathy. Diabetic retinopathy is a leading cause of blindness and visual disability. Research findings suggest that, after 15 years of diabetes, approximately 2% of people become blind, while about 10% develop severe visual handicap (Global Burden of Diabetes, WHO).

Although the underlying causes of hyperglycemia are multiple, a common thread associated with high levels of blood sugar is the development of a range of vascular and inflammatory complications that might seriously limit the quality and duration of life in affected individuals (Schmidt AM & Stern DM, Trends Endocrinol Metab.2000 Nov;11(9):368-75) Mediators of vascular damage of diabetes include poor glycemic control, lipoprotein abnormalities, hypertension, oxidative stress, inflammation and advanced glycation end-products (AGEs). AGE accumulation causes arterial stiffening in the vessel wall, glomerulosclerosis in the kidney, and vascular hyperpermeability in the retina. Through their interaction with their putative receptor the so-called receptor for AGEs (RAGE), AGEs activate endothelial cells and macrophages, generate reactive oxygen species (ROS), induce overexpression of vascular endothelial growth factor (VEGF) and vascular cell adhesion molecule-1 (VCAM-1), and quench nitric oxide (NO) (Soro-paavonen A & Forbes JM, Curr Med Chem. 2006;13(15):1777-88).

Current drugs used for managing diabetes and its precursor syndromes, such as insulin resistance, generally fall within five classes of compounds: biguanides, thiazolidinediones, sulfonylureas, meglitinides and alpha-glucosidase inhibitors.

Treatment of diabetic complications such as diabetic macroangiopathy, diabetic microangiopathy, diabetic neuropathy, diabetic nephropathy, diabetic retinopathy, foot ulcers, myocardial infarction, or diabetic cardiomyopathy are generally treated by using current treatment strategies directed at slowing the progression of diabetic nephropathy or other diabetic complications using various approaches, including optimized glycemic control (through modification of diet and/or insulin therapy) and hypertension control. These therapeutic strategies have demonstrated varying degrees of success.

For example, both angiotensin-converting enzyme (ACE) inhibitors and angiotensin receptor blockers (ARBs), administered to reduce hypertension, have been shown to delay progression or development of nephropathy and macroalbuminuria. Further, while glycemic and blood pressure control therapies significantly decrease the morbidity and mortality associated with diabetic nephropathy by delaying progression of associated pathologies, such conventional therapies do not adequately halt the progression of the disease and thus fail to provide a complete therapeutic effect. In addition, administration of ACE inhibitors or ARBs, the current standard of care, are not universally effective and only minimally delay the progression of nephropathy.

Thus, there is a critical need for better drugs and/or combination of approach since current treatment may have limited impact on the progressive complications. Hypertension affects large number of population worldwide and it is estimated that by 2025 the global prevalence may set to rise to 29.2% of all adults - a total of between 1.54 and 1.58 billion individuals (Kearney PM et al., Lancet 2005; 365(9455): 217-23). Prevalence may set to increase by 24% in developed countries and by 80% in developing countries, so that in 20 years' time, three quarters of the world's population with hypertension would live in developing countries (Karen TM et al. CMAJ, 178(11); 1429-35).

Currently available therapy includes diuretics such as hydrochlorothiazide, cicletanine, xipamide, indapamide, clopamide, amiloride, spironolactone and canrenone; beta-blockers such as propranolol, acebutolol, atenolol, nadolol, bisoprolol, metoprolol, pindolol, oxprenolol and betaxolol; ACE inhibitors such as captopril, enalapril, benazepril, lisinopril, quinapril, ramipril and imidapril; Angiotensin II receptor antagonists (ARBs), such as losartan, candesartan, cilexetil, irbesartan, telmisartan, and valsartan; calcium channel antagonists, such as nifedipine, amlodipine, felodipine, isradipine, diltiazem, bepridil, lacidipine, nitrendipine, nicardipine and verapamil; central anti-hypertensive agents such as clonidine, guanfacine, monoxidine, rilmenidine and α-methyl-dopa; alpha-blockers such as prasozin, urapidil, doxazosine and terazosine; Vasodilators such as hydralazine, dihydralazine and minoxidil.vasopeptidase inhibitors; Endothelin antagonists and rennin inhibitors such as aliskiren.

It has generally been observed that in a long term treatment monotherapy with any of the above drug does not adequately control the symptoms of hypertension and patients ultimately progress toward various complication of hypertension. This happens due to the complexity of disease process and involvement of different factors causing hypertension. The precise pathophysiological mechanisms through which elevated blood pressure leads to cardiovascular disease, however, remain uncertain. Basic data suggest that increasing levels of blood pressure may stimulate a proinflammatory response and that endothelial inflammation may also herald the changes in arterial wall that characterize the hypertensive state. (Blake GJ et al. Circulation, 2003;108:2993-2999.) . Patients with cardiovascular disease present with increase expression and plasma concentration of inflammatory markers and mediators, which include CRP (C-reactive protein) and adhesion molecules, such as selectins (P-selectin, E-selectin and L-selectin), ICAM-1 (Intracellular adhesion molecule-1) and VCAM-1(vascular cell adhesion molecule-1). Moreover, increased plasma levels of primary inflammatory cytokine TNF-alpha(Tumour necrosis factor-alpha), and the secondary inflammatory cytokine IL9Interleukin)-6 as well as ICAM-1, VCAM-1, E-selectin, vWF(von Willebrand factor) and CRP, have been demonstrated in patients with hypertension. High levels of inflammatory mediators, particularly IL-6, ICAM-1 and CRP, may be independent risk factors for the development of hypertension. They may also be associated with increased risk of diabetes.(Savoia C & Schiffrin EL, Clinical Science (2007); 112: 375-84) Clinicians normally tackle this problem using different combination of drugs. A combination treatment increases the number of mechanisms potentially capable of reducing an elevated blood pressure, future complications and reduces the rate and magnitude of the adverse events produced by each drug. Further, the addition of one agent may counteract some deleterious effect of the other. Additionally, a number of patients are either nonresponsive to one or more of the available monotherapies. However it has been observed that even after combining different drugs maintaining the symptomatic control and preventing its complications for longer period still remains difficult.

Heart failure constitutes a major public health burden in the western world. Since incidence rates appear to remain stable over the years, at least in men, prevalence estimates of heart failure are bound to increase as the population ages. Hospitalisation rates for heart failure have increased considerably. The proportion of patients having multiple hospital admissions is rising. Heart failure continues to be a fatal disease, despite advances in treatment, with only 35% surviving 5 years after the first diagnosis. Prevention of the development of heart failure in high-risk patients is therefore fundamental (Bleumink G et al. European Heart Journal, 2004; 25: 1614-1619). As the leading cause of hospitalization for individuals aged 65 years and older, congestive heart failure (CHF) is emerging as a major public health concern. The CHF problem is magnified in individuals with diabetes, in whom incidence rates are two to five times greater than those in the general population. Nonetheless, heart failure has recently been termed "the frequent, forgotten, and often fatal complication of diabetes" , in part because estimates of the association between diabetes and CHF have been established primarily in studies that include diabetes as a potential risk factor in general populations (Nichols G et al. Diabetes Care, 2004, 27: 1879-1884). Hence, while therapies for heart disease have greatly improved and life expectancies have extended over the last several years, new and better therapies continue to be sought.

Although, presently used treatments can alleviate symptoms of CHF and correct certain pathophysiologic abnormalities caused by the disease process, CHF remains a relentlessly progressive condition with a relatively high rate of mortality. In fact, relative reductions in morbidity and mortality brought about by existing drugs are on the order of about 10 to 25 percent. Drug therapies, using known active ingredients such as vasodilators, angiotensin II receptor antagonists, ACE inhibitors, diuretics, antithrombolytic agents, β-adrenergic receptor antagonists, α-adrenergic receptor antagonists, calcium channel blockers, and the like, are available for treating heart failure and associated diseases.

Accumulating evidence indicates that inflammatory mediators are important in the pathogenesis of chronic heart failure (CHF), contributing to cardiac remodeling and peripheral vascular disturbances. Several studies have shown increased levels of inflammatory cytokines, such as tumor necrosis factor-alpha (TNF-alpha), interleukin (IL)-1beta, and IL-6 in patients with CHF in both plasma and circulating leukocytes as well as in the failing myocardium itself. Importantly, this increase in inflammatory mediators does not seem to be accompanied by a corresponding increase in antiinflammatory cytokines, such as IL-10 and transforming growth factor-beta, resulting in an inflammatory imbalance in the cytokine network. (Gullestad L & Aukrust P, Am J Cardiol. 2005 Jun 6; 95(11A):17C-23C; discussion 38C-40C) Since heart failure is a clinical syndrome arising from diverse causes and is accompanied by adverse changes in physiological function of organs other than the heart, the appropriate selection of therapeutic agents to yield improvements in cardiovascular morbidity and survival at the various stages of cardiovascular disease frequently requires the concurrent administration of drugs from several classes of therapeutic agents, such as ACE inhibitors, angiotensin-receptor antagonists, beta-blockers, HMG CoA reductase inhibitors (statins), and aldosterone antagonists.

These diseases are multifactorial having varied etiopathology and thus most of the time response to monotherapy does not become sufficient and gradually diminishes.

Major advantages of combination therapy:
1. More than one cause of action can be targeted and thus complimentary actions can be achieved.
2. Due to synergistic effects of combination low dose of each drug can be administered as compared to monotherapy.
3. Few side effects due to low individual dose
4. Reduces the possibilities of complications

WO 2006/002983 discloses a pharmaceutical combination comprising an AT1 receptor blocker or pharmaceutically acceptable salts thereof, particularly valsartan and a compound which exhibits advanced glycosylation end product (AGE) breaking activity (also called AGE breaker)or a pharmaceutically effective salts thereof.

However, in spite of different modalities of available treatment, there exists a need for better therapeutic approaches, which effectively control the symptoms of these diseases as well as reduces the possibilities or delay the complications.

It has been surprisingly found that compounds of formula (I) and (II) are effective in the treatment of cardiovascular complications such as heart failure more particularly diabetes induced heart failure. Moreover, inventors of present invention have found that the compounds of formula (I) and/or (II), which has AGE inhibitory, AGE breaking and free radical scavenging effect, when co used with the currently available therapy, better symptomatic control can be achieved and quality of life can be improved.

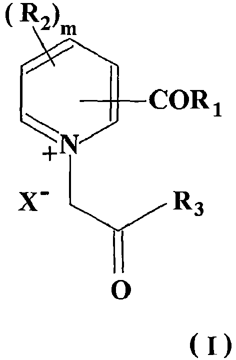

R₁, R₂, R₃, X and m of formula (I) are as defined hereinafter.

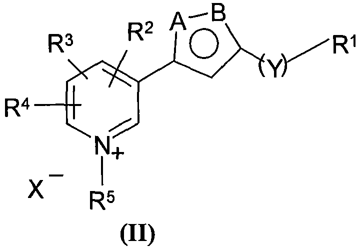

_{R}¹, R², _{R}³, _{R}⁴, R⁵, X, Y, A and B of formula (II) as defined hereinafter.

### Summary of the Invention:

In one embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure; or a pharmaceutically acceptable salts thereof, optionally in presence of a pharmaceutically acceptable carrier.

In a preferred embodiment, the present invention relates to a pharmaceutical combination or composition comprising: (a) compound of formula (I) and/or (II) as defined above or a pharmaceutically acceptable salt thereof; (b) at least one antihypertensive agent or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt there of; (b) one or more antihypertensive agent; wherein said antihypertensive agent include but not limited to an angiotensin converting enzyme (ACE) inhibitor, a renin inhibitor, a beta adrenergic receptor blocker, an alpha adrenergic receptor blocker, a calcium channel blocker, a potassium channel activator, an aldosterone synthase inhibitor, a neutral endopeptidase (NEP) inhibitor, a dual angiotensin converting enzyme/neutral endopeptidase (ACE/NEP) inhibitor, an endothelin receptor antagonist, a dual angiotensin and endothelin receptor antagonist (DARA), a diuretic or a pharmaceutically acceptable salt thereof, optionally in the presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an angiotensin converting enzyme (ACE) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a renin inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a beta adrenergic receptor blocker or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an alpha adrenergic blocker or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a calcium channel blocker or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a potassium channel activator or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a dual angiotensin and endothelin receptor antagonist (DARA) or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an aldosterone synthase inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a neutral endopeptidase (NEP) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an endothelin receptor antagonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a diuretic or a pharmaceutically acceptable salt thereof, optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of hypolipidemic agent or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) one or more hypolipidemic agent; wherein said hypolipidemic agent include but not limited to an MTP inhibitor, a HMG CoA reductase inhibitor, a squalene synthetase inhibitor, a fibric acid derivative, an ACAT inhibitor, lipoxygenase inhibitors, a cholesterol absorption inhibitor, an ileal Na +/bile acid cotransporter inhibitor, upregulators of LDL receptor activity, a cholesteryl ester transfer protein(CETP) inhibitor, a bile acid sequestrant, and/or a nicotinic acid and derivative, or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an MTP inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a HMG CoA reductase inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a squalene synthetase inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a fibric acid derivative or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an ACAT inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a cholesterol absorption inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an ileal Na +/bile acid cotransporter inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a cholesteryl ester transfer protein(CETP) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a bile acid sequestrant or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a nicotinic acid and its derivative or a pharmaceutically acceptable salt thereof, optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of antidiabetic agent or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) one or more antidiabetic agent; wherein said antidaibetic agent include but not limited to a biguanide such as metformin, phenformin, a sulfonylurea such as gliclazide, an alpha glucosidase inhibitor, a PPARγ agonist such as thiazolidinediones, a PPARα agonist such as fibric acid derivatives, an alpha-amylase inhibitor, a fatty acid oxidation inhibitor, an A2 antagonist, a PPARδ agonist or antagonist, a PPARα/γ dual agonist, an aP2 inhibitor, a dipeptidyl peptidase IV (DP4) inhibitor, a SGLT2 inhibitor, a glycogen phosphorylase inhibitor, a glucagon-like peptide-1 (GLP-1), a glucokinase activator, a VPAC2 receptor agonist, a PTP-1B (protein tyrosine phosphatase-1B) inhibitor, an 11β-HSD 1 (11β-hydroxy-steroid dehydrogenasel) inhibitor or meglitinide, as well as insulin, or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a biguanide or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a sulfonylurea or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an alpha glucosidase inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a PPARγ agonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a PPARα agonist or antagonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a PPARδ agonist or antagonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a PPARα/γ dual agonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an aP2 inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a dipeptidyl peptidase IV (DP4) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a SGLT2 inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a glycogen phosphorylase inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a glucagon-like peptide-1 (GLP-1) or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a PTP-1B (protein tyrosine phosphatase-1B) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) an 11β-HSD 1 (11β-hydroxy-steroid dehydrogenase 1) inhibitor or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a meglitinide or a pharmaceutically acceptable salt thereof, optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of antiobesity agent or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) one or more antiobesity agent; wherein said antiobesity agent include but not limited to a 5HT (serotonin) transporter inhibitor, a NE (norepinephrine) transporter inhibitor, a CB-1 (cannabinoind-1 receptor) antagonist/inverse agonist, a ghrelin antibody, a ghrelin antagonist, a H3 (histamine H3) antagonist/inverse agonist, a NPY1 (neuropeptide Y Y1) antagonist, a NPY2 (neuropeptide Y Y2) agonist, a NPY5 (neuropeptide Y Y5) antagonist, a leptin or its derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a β3 (beta adrenergic receptor 3) agonist, a DGAT1 (diacylglycerol acyltransferase 1) inhibitor, a DGAT2 (diacylglycerol acyltransferase 2) inhibitor, a FAS (fatty acid synthase) inhibitor, a PDE (phosphodiesterase) inhibitor, a thyroid hormone β agonist, an UCP-1 (uncoupling protein 1), 2,. or 3 activator, an acyl-estrogen, a glucocorticoid antagonist, a SCD-1 (stearoyl-CoA desaturase-1) inhibitor, a lipase inhibitor, a fatty acid transporter inhibitor, a dicarboxylate transporter inhibitor, or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a CB-1 antagonist/inverse agonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a Neuropeptide (NPY1/NPY5) antagonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a beta.3 adrenergic receptor (beta 3) agonist or a pharmaceutically acceptable salt thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) a thyroid hormone beta agonist or a pharmaceutically acceptable salt thereof, optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of antiplatelet and antithrombotic agent or a pharmaceutically acceptable salts thereof, optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of agent for diabetic vascular complications or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) one or more agent for diabetic vascular complications; wherein said agent include but not limited to an aldose reductase inhibitor, an AGE inhibitor or an AGE breaker or a pharmaceutically acceptable salt thereof optionally in presence of a pharmaceutically acceptable carrier.

In an another preferred embodiment, the present invention relates to a pharmaceutical combination or composition comprising: (a) compound of formula (I) and/or (II) as defined above or a pharmaceutically acceptable salt thereof; (b) at least one therapeutic agent selected from the group consisting of an agent used for the treatment of heart failure or a pharmaceutically acceptable salts thereof, optionally in presence of a pharmaceutically acceptable carrier.

In a further embodiment, the present invention relates to a pharmaceutical combination or composition comprising: (a) compound of formula (I) and/or (II) as defined above or a pharmaceutically acceptable salt thereof; (b) one or more agent used for the treatment of heart failure; such agent include but not limited to a digitalis glycoside, a phosphodiesterase inhibitor or a pharmaceutically acceptable salt thereof optionally in presence of a pharmaceutically acceptable carrier.

In one another preferred embodiment, the present invention relates to a pharmaceutical combination or composition comprising: (a) compound of formula (I) and/or (II) as defined above or a pharmaceutically acceptable salt thereof; (b) (i) an antihypertensive agent; and (ii) one or more drugs selected from the group consisting of: a diuretic; an antidiabetics; a hypolipidemic; an antiplatelet; and an antithrombotic agent; or a pharmaceutically acceptable salts thereof optionally in presence of a pharmaceutically acceptable carrier.

In one another preferred embodiment, the present invention relates to a pharmaceutical combination or composition comprising: (a) compound of formula (I) and/or (II) as defined above or a pharmaceutically acceptable salt thereof; (b) (i) an antidiabetic agent and (ii) one more drugs selected from the group consisting of: a diuretic; an antihypertensive; a hypolipidemic; an antiplatelet; and an antithrombotic agent; or a pharmaceutically acceptable salts thereof optionally in presence of a pharmaceutically acceptable carrier.

In one another preferred embodiment, the present invention relates to a pharmaceutical combination or composition comprising: (a) compound of formula (I) and/or (II) as defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier.

In present invention each component such as (a), (b), (c) & (d) can be administered together, one after the another or separately in one combined unit dosage form or in separate unit dosage form. The unit dosage form may also be a fixed combination.

In another embodiment, the present invention relates to a method of treating heart failure comprising administering a therapeutically effective amount of compound of formula (I) and/or (II) or a pharmaceutically acceptable salts thereof.

In another preferred embodiment, the present invention relates to a method of treating heart failure associated with diabetes comprising administering a therapeutically effective amount of compound of formula (I) and/or (II) or a pharmaceutically acceptable salts thereof.

In another embodiment the invention provides the use of compound of formula (I) and/or (II) or a pharmaceutically acceptable salts thereof for treatment of heart failure. In another embodiment the invention provides the use of compound of formula(I) and/or (II) or a pharmaceutically acceptable salts thereof for treatment of heart failure associated with diabetes.

In one another preferred embodiment, the present invention relates to method of an administration of pharmaceutical combination or composition comprising: (a) compound of formula (I) and/or (II) as defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier, for the treatment or prevention of the disease condition related to diabetes or aging-related vascular complications, preferably congestive heart failure and more preferably diabetes associated congestive heart failure.

In one another preferred embodiment, the present invention relates to the use of pharmaceutical combination or composition comprising: (a) compound of formula (I) and/or (II) as defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier, for the treatment or prevention of the disease condition related to diabetes or aging-related vascular complications, preferably congestive heart failure and more preferably diabetes associated congestive heart failure.

In another embodiment, the present invention relates to a method of treating or preventing the disease condition related to diabetes or aging-related vascular complications, such as such as neuropathy (both diabetic and non-diabetic), nephropathy (both diabetic and non-diabetic), diabetic retinopathy, diabetic cardiomyopathy, hypertension, hypertensive cardiomyopathy, and dilated cardiomyopathy by administration of a therapeutically effective amount of any pharmaceutical combination or composition according to the present invention comprising: (a) a compound of formula (I) and/or (II) as defined above or its pharmaceutically acceptable salts thereof and (b) at least one therapeutic agent selected from the group consisting of: an antihypertensive agent; an antidiabetics agent; a hypolipidemic agent; an antiplatelet agent; an antiobesity agent; an antithrombotic agent; an agent for diabetic vascular complications; and an agent for treatment of heart failure or a pharmaceutically acceptable salts thereof, optionally in presence of a pharmaceutically acceptable carrier to a mammal in need thereof.

In another embodiment the invention provides the use of pharmaceutical combination or composition of the instant invention comprising: (a) a compound of formula (I) and/or (II) as defined above or its pharmaceutically acceptable salts thereof and (b) at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure or a pharmaceutically acceptable salts thereof, optionally in presence of a pharmaceutically acceptable carrier, to treat or prevent diabetes and aging-related vascular complications, such as neuropathy (both diabetic and non-diabetic), nephropathy (both diabetic and non-diabetic), diabetic retinopathy, diabetic cardiomyopathy, hypertension, hypertensive cardiomyopathy, and dilated cardiomyopathy to a mammal in need thereof.

In another embodiment the present invention relates to methods of treating or preventing the disease condition selected from the group consisting of hypertension, congestive heart failure of diverse etiology like left ventricular dysfunction (systolic and diastolic), ischemic cardiomyopathy, hypertrophic cardiomyopathy, diabetic cardiomyopathy, hypertensive cardiomyopathy, dilated cardiomyopathy and metabolic cardiomyopathy (thyroid, carcinoid, pheochromocytoma or acromegaly associated), myocardial infarction and its sequelae, atherosclerosis (and complications thereof), angina (whether unstable or stable), renal insufficiency (diabetic and non-diabetic), renal failure conditions, such as diabetic nephropathy, hypertensive nephropathy, and neuropathy (both diabetic and non-diabetic), by administration of a therapeutically effective amount of any pharmaceutical combination or composition according to the present invention comprising: (a) a compound of formula (I) and/or (II) as defined above or its pharmaceutically acceptable salts thereof plus (b) at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure or a pharmaceutically acceptable salts thereof, optionally in presence of a pharmaceutically acceptable carrier to a mammal in need thereof.

In another embodiment the invention provides the use of pharmaceutical combination or composition of the instant invention comprising (a) a compound of formula (I) and/or (II) as defined above or its pharmaceutically acceptable salts thereof plus (b) at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure; or a pharmaceutically acceptable salts thereof, optionally in the presence of a pharmaceutically acceptable carrier, to treat or prevent the disease condition selected from the group consisting of hypertension, congestive heart failure of diverse etiology like left ventricular dysfunction (systolic and diastolic), ischemic cardiomyopathy, hypertrophic cardiomyopathy, diabetic cardiomyopathy, hypertensive cardiomyopathy, dilated cardiomyopathy and metabolic cardiomyopathy (thyroid, carcinoid, pheochromocytoma or acromegaly associated), myocardial infarction and its sequelae, atherosclerosis (and complications thereof), angina (whether unstable or stable), renal insufficiency (diabetic and non- diabetic), renal failure conditions, such as diabetic nephropathy, hypertensive nephropathy, and neuropathy (both diabetic and non-diabetic), to a mammal in need thereof.

The present invention also provides a kit comprising in separate containers in a single package pharmaceutical compositions comprising in one container a pharmaceutical composition comprising a compound of formula (I) and/or (II) as defined above and in a second container a pharmaceutical composition comprising at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure or a pharmaceutically acceptable salts thereof. The kit form is particularly useful when the each components are required to be administered in different dosage forms and/or at different dosage intervals. There can be more such containers as per requirements.

### Detailed Description:

The present invention relates to a combination, such as a combined preparation or pharmaceutical composition comprising: (a) compound of formula (I) and/or (II) as defined above or a pharmaceutically acceptable salt thereof (b) at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure; or a pharmaceutically acceptable salts thereof, optionally in presence of a pharmaceutically acceptable carrier for separate, simultaneous or sequential use. 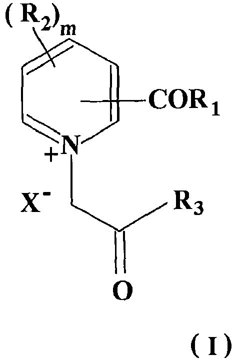 wherein
R₁ is -R₄-R₅ or -N(R₇) N (R₇) R₉;
R₄ is selected from the group consisting of -N(R₇)R₆O-, -N(R₇)R₆N(R₇)-, OR₆O,
and -OR₆N(R₇)-, where R₆ is alkyl with C₂ to C₈ carbon atoms; R₅ is selected from the group consisting of alkyl, aryl including heteroaryl, -COR₇, SO₂R₇, -C(S) NHR₇,-C(NH)NHR₇, -COR₁₀, 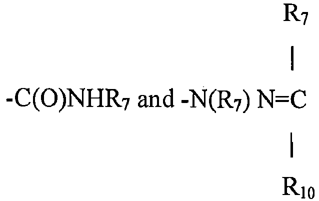 where R₇ is selected from the group consisting of H, alkyl and aryl including heteroaryl provided R₇ may be the same or different for R₁ and R₃ in the same compound;
R₂ is selected from the group consisting of F, Cl, Br, I, OR₇, NO₂, alkyl, aryl including heteroaryl, formyl, acyl, C(O)NR₇R₁₀, C(O)OR₇, NR₇R₁₀, N=C(R₇)(R₁₀), SR₇, SO₂NH₂, SO₂ alkyl and SO₂aryl,
and m is 0, 1 or 2;
R₃ is selected from the group consisting of R₇, OR₇, N(R₇) (R₁₀), N=C(R₇) (R₁₀), N(R₇) N(R₇) (R₁₀), N(R₇) N=C(R₇) (R₁₀) and CH(R₇)C(O)R₈;
where R₈ is selected from the group consisting of R₇, OR₇ and NR₇R₁₀;
R₉ is selected from the group consisting of hydrogen, alkyl, aryl including heteroaryl, C(O)R₁₀, -SO₂R₁₀, -C(S)NHR₁₀, -C(NH) NH (R₁₀) and -C(O) NHR₁₀,
R₁₀ is selected for the group consisting of H, alkyl or aryl including heteroaryl and in each case may be the same or different from substituent R₇, provided R₁₀ may be the same or different for R₁ and R₃ in the same compound;
X is selected from group consisting of a halide ion, acetate ion, perchlorate ion,
sulfonate ion, oxalate ion, citrate ion, tosylate ion, maleate ion, mesylate ion,
carbonate ion, sulfite ion, phosphoric hydrogen ion, phosphonate ion, phosphate ion,
BF₄⁻ and PF₆⁻ or null;
with proviso that,
(i) when two alkyl groups are present on the same carbon or nitrogen, they may be linked together to form a cyclic structure ;
(ii) the nitrogen of heteroaryl ring of R₁₀, when present, may be quaternized;
(iii) when R₃ is OR₇ and R₁ is -NHNH₂ then R₇ is not alkyl and
(iv) when R₃ is OR₇, R₁ is N(R₇)(NR₇)R₉ and R₉ is C(O)R₁₀ where
R₁₀ is alkyl, then R₇ is not hydrogen.

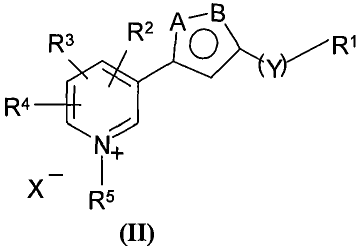

Wherein R¹ is alkyl or aryl group;
Y is selected from the group comprising of sulfur, oxygen, nitrogen, or alkylene;
A and B are independently selected from nitrogen, NH, NR6, sulfur, oxygen or carbon to form heteroaromatic ring system;
R², R³ and R⁴ are independently selected from the group consisting of F, Cl, Br, I, OR⁷, NO₂, alkyl, aryl including heteroaryl, formyl, acyl, C(O)NR⁶R⁷, C(O)OR⁶, NR⁶R⁷, N=C(R⁶)(R⁷), SR⁶, S0₂NH₂, SO₂alkyl, SO₂aryl; R², R³ and R⁴ might be optionally joined together to form a ring system;
R⁵ is independently selected for the group consisting of alkyl, aryl and null;
R⁶ is independently selected from the group consisting of H, alkyl and aryl including heteroaryl provided R⁶ might be different for R², R³ and R⁴ in the same compound;
R⁷ is independently selected from the group consisting of H, alkyl and aryl including heteroaryl and in each case optionally different from substituent R⁶, provided R⁷ might be different for R², R³ and R⁴ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogenion; phosphonate ion, phosphate ion, BF4, PF6 and null;
with proviso that when two alkyl groups are present on the same carbon or nitrogen, they are optionally linked together to form a cyclic structure.

As used herein, "alkyl" refers to an optionally substituted hydrocarbon group joined by single carbon-carbon bonds and having 1 to 8 carbon atoms joined to gather. The alkyl hydrocarbon group may be linear, branched or cyclic, saturated or unsaturated. The substituents are selected from F, Cl, Br,I, N, S,O and aryl. Preferably, no more than three substituents are present.

As used herein "aryl" refers to an optionally substituted aromatic group with atleast one ring having a conjugated pi-electron system, containing upto two conjugated or fused ring systems. Aryl includes carbocyclic aryl, heterocyclic aryl and biaryl groups, all of which may be optionally substituted. The substituents are selected from F, Cl, Br, I, N, S, O and straight chain or branched C₁-C₆ hydrocarbon.
The compounds as per formula I and II can be prepared as per the process described in WO/01/25208 and WO/02/85897.

The following list of compounds provides representative compounds of the general formula (I) and (II):
1-(2-ethoxy -2- oxoethyl) -4-(phenylsulfonyl hydrazino carbonyl) pyridinium bromide (compound 1);
1-(2-phenylamino-2-oxoethyl)-4- (phenylsulfonyl hydrazino carbonyl) pyridinium chloride (compound 2);
1-(2-ethoxy -2- oxoethyl) -3-(phenylsulfonyl hydrazino carbonyl) pyridinium bromide (compound 3);
1-(2-(2',4'-dichlorophenyl)-2-oxoethyl)-3-(2(methoxy)ethyloxycarbonyl) pyridinium bromide (Compound 4);
1-(2-phenylamino-2-oxoethyl)-3-((benzoyloxy)ethylaminocarbonyl) pyridinium chloride (compound 5);
1-(2-thien-2'-yl- 2-oxoethyl)-3-(phenylaminocarbonyl hydrazinocarbonyl)pyridinium bromide (compound 6);
1-(2-phenyl-2-oxoethyl)-3-(2-(acetoxy)ethylaminocarbonyl) pyridinium bromide (compound 7);
1-(2-phenylamino-2-oxoethyl)-3-(phenyl sulfonyl hydrazino carbonyl) pyridinium chloride (compound 8);
1-(2-phenylamino-2-oxoethyl)-3-((4-methylphenyl) sulfonyl hydrazino carbonyl)pyridinium chloride (compound 9);
1-(2-phenyl-2-oxoethyl)-3-(2-(benzoyloxy)ethyloxy carbonyl) pyridinium bromide (compound 10);
1-(2-thien-2'-yl-2-oxoethyl)-3-(phenylcarbonyl hydrazino carbonyl) pyridinium bromide (compound 11);
1-(2-ethoxy-2-oxoethyl)-3-((phenylmethyl)sulfonyl hydrazino carbonyl)pyridinium bromide (compound 12);
1-(2-phenyl-2-oxoethyl)-3-((phenylmethyl)sulfonyl hydrazino carbonyl) pyridinium bromide (compound 13);
N, N' - bis [3-carbonyl-1-(2-furan-2'-yl-2-oxoethyl) pyridinium] hydrazine dibromide. (Compound No: 14);
N, N'-bis [3-carbonyl-1- (2-thien-2'-yl-2-oxoethyl) pyridinium] hydrazine dichloride.(Compound No: 15);
1-(2-thien-2'-yl-2-oxoethyl)-3-((2-(1-oxo-3-cyclohexyl)-propyl)-hydrazino carbonyl)-pyridinium bromide.(Compound No: 16);
1-(2-phenylamino-2-oxo ethyl)-3-({2-(-oxo-3-cyclohexy)-propyl}-hydrazinocarbonyl}-pyridinium bromide.(Compound No: 17);
1-(2-thien-2'-yl-2-oxoethyl)-3-[2-(benzoyloxy)ethylamino carbonyl]-pyridinium bromide (Compound No: 18);
1-(4-ethoxy-2,4-dioxobutyl)-3-(2-(benzoyloxy)ethylamino carbonyl)-pyridinium chloride. (Compound No: 19);
1-(2', 4'-dichloro-phenyl-2-oxoethyl)-3-(2-methoxyethyl aminocarbonyl)-pyridinium bromide. (Compound No: 20);
N,N'-bis-[3-carbonyl-1-(2-cyclopropylamino-2-oxoethyl) pyridinium] hydrazine dichloride. (Compound No: 21);
1-(2-cyclopropylamino-2-oxoethyl)-3-(2-methoxyethylaminocarbonyl)-pyridinium chloride. (Compound No: 22);
N-N'-bis [3-carbonyl-1-(2-isopropylamino-2-oxoethyl) pyridinium] hydrazine dichloride. (Compound No: 23);
1-(2-thien-2'yl-2-oxoethyl)-3-(2-(2-chloro-3-pyridoylhydrazinocarbonyl) -pyridinium chloride. (Compound No: 24);
1-(2-isopropylamino-2-oxoethyl)-3-(2-methylsulfonylhydrazinocarbonyl) -pyridinium chloride. (Compound No: 25);
1-(2-(1-pyrrolidinyl)-2-oxoethyl)-3-(methanesulfonyl hydrazino carbonyl) pyridinium chloride. (Compound No: 26);
1-(2-thien-2'-yl-2-oxoethyl)-3-(methanesulfonyl hydrazino carbonyl) pyridinium chloride. (Compound No: 27);
N,N'-bis [3 -carbonyl-1 -(2-hydroxy-2-oxoethyl)pyridinium]hydrazine dichloride (Compound No: 28);
1-(2-thien-2'-yl-2-oxoethyl)-3-((2-methoxy ethyl) amino carbonyl)-5-bromo pyridinium chloride (Compound No: 29);
1-(2'-thien-2'-yl-2-oxoethyl)-3-[1-oxo-1-(2-methoxy carbonyl)pyridyl] hydrazino pyridinium chloride. (Compound No: 30);
1-[1-(2-thien-2'-yl-2-oxoethyl)-6-methyl-3-carbonyl pyridinium]-2-[1-(2-Thien-2'-yl-2-oxoethyl)-3-carbonyl pyridinium] hydrazine dichloride(compound no: 31);
1-(2-thien-2'-yl-2-oxoethyl)-3-(isopropylsulfonyl hydrazino carbonyl) pyridinium bromide(compound no: 32);
1-(2-(4-benzyl piperidin-1-yl)-2-oxoethyl)-3-(methanesulfonyl hydrazino carbonyl) pyridinium chloride(compound no: 33);
1-(2-(2-ethoxy carbonyl pyrrolidin-1-yl)-2-oxoethyl)-3-(methanesulfonyl hydrazino carbonyl) pyridinium chloride. (compound no: 34);
1-(2-thien-2'-yl-2-oxoethyl)-3-(methanesulfonyl hydrazino carbonyl)-5-bromo pyridinium bromide. (compound no: 35);
1-(2-thien-2'-yl-2-oxoethyl)-3-(ethoxycarbonyl hydrazino carbonyl) pyridinium bromide. (compound no: 36);
1-(2-(5-chloro-thien-2-yl)-2-oxoethyl)-3-(methanesulfonyl hydrazino carbonyl) pyridinium bromide (compound no: 37);
N,N'-bis[3-carbonyl-1-(2-(4-nitro-thien-2-yl)-2-oxoethyl)pyridinium] hydrazine dichloride. (compound no: 38);
1-(2-thien-2'-yl-2-oxoethyl)-3-(methanesulfonyl hydrazino carbonyl) -6-methyl pyridinium bromide. (compound no: 39);
N,N'-bis[3-carbonyl-1-(2-(5-methyl-thien-2-yl)-2-oxoethyl) pyridinium] hydrazine dichloride. (compound no: 40);
N,N'-bis[3-carbonyl-1-(2-(2-ethoxycarbonyl pyrrolidin-1-yl)-2-oxoethyl) pyridinium] hydrazine dichloride. (compound no: 41);
1-[1-(2-thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 42);
1-(2-(4-carbethoxy-thiazolidin-3-yl)-2oxoethyl)-3-(methanesulfonyl hydrazino carbonyl) pyridinium chloride (compound no: 43);
N,N'-bis[3-carbonyl-1-(2-(5-chloro-thien-2-yl)-2-oxoethyl) pyridinium] hydrazine dichloride. (compound no: 44);
1-(2-(5-methyl-thien-2-yl)-2-oxoethyl)-3-(methanesulfonyl hydrazino carbonyl) pyridinium chloride (compound no: 45);
1-(2-(4-nitro-thien-2-yl)-2-oxoethyl)-3-(methanesulfonyl hydrazino carbonyl) pyridinium bromide. (compound no: 46);
1-(2-phenylamino-2-oxoethyl)-3-(phenyl hydrazino carbonyl) pyridinium chloride (compound no: 47);
1-(2-phenylamino-2-oxoethyl)-4 -[2-(benzoyloxy) ethylamino carbonyl] pyridinium chloride (compound no: 48);
1-2-(5-nitro-thien-2-yl)-2-oxoethyl)-3-(methanesulfonyl hydrazino carbonyl) pyridinium chloride (compound no: 49);
1-(2-thien-2'-yl-2-oxoethyl)-3-(trifluoromethanesulfonyl hydrazino carbonyl) - pyridinium bromide. (compound no. 50);
1-(2-thien-2'-yl-2-oxoethyl)-3-(phenyl hydrazino carbonyl) pyridinium bromide (compound no.51);
1-(2-thien-2'-yl-2-oxoethyl)-3-(p-methoxy phenyl sulfonyl hydrazino carbonyl) pyridinium bromide (compound no. 52);
1-(2-ethoxy-2-oxoethyl)-3-(phenyl aminocarbonyl hydrazino carbonyl) pyridinium bromide (compound no. 53);
1-(2-ethoxy-2-oxoethyl)-3-(p-toluene sulfonyl hydrazino carbonyl) pyridinium bromide (compound no. 54);
1-(2-phenyl-2-oxoethyl)-3-(phenylamino carbonyl hydrazino carbonyl) pyridinium bromide (compound no. 55);
1-(2-phenylamino-2-oxoethyl)-3-(benzyl sulfonyl hydrazino carbonyl) pyridiniumchloride. (compound no. 56);
1-(2-phenyl-2-oxoethyl)-4-(methanesulfonyl hydrazino carbonyl) pyridinium bromide (compound no. 57);
1-(2-phenyl-2-oxoethyl)-3-(phenyl hydrazino carbonyl) pyridinium bromide. (compound no. 58);
1-(2-ethoxy-2-oxoethyl)-4-[2-(benzoyloxy) ethyl amino carbonyl] pyridinium bromide (compound no. 59);
1-(2-ethoxy-2-oxoethyl)-3-(phenyl hydrazino carbonyl) pyridinium bromide. (compound no. 60);
1-(2-phenyl-2-oxoethyl)-3-(p-methoxyphenyl sulfonyl hydrazino carbonyl) pyridinium bromide. (compound no. 61);
1-(2-phenyl-2-oxoethyl)- 4-[2-(benzoyloxy) ethyl amino carbonyl] pyridinium bromide. (compound no. 62);
1-(2-ethoxy-2-oxoethyl)- 4-(p-methanesulfonyl hydrazino carbonyl) pyridinium bromide. (compound no. 63);
N, N'-Bis [3-carbonyl-1- (2-phenyl-2-oxoethyl)-pyridinium] hydrazine dibromide (compound 64);
N, N'-Bis [3-carbonyl-1-(2-ethoxy-2-oxoethyl) pyridinium] hydrazine dibromide (compound 65);
N, N'-Bis [3-carbonyl-1- (2- (2, 4-dichlorophenyl)-2-oxoethyl) pyridinium] hydrazine dibromide (compound 66);
1- (2-Ethoxy-2-oxoethyl)-3- (2- (2-pyridyl) hydrazinocarbonyl) pyridinium bromide (compound 67);
1- (2-Thien-2'-yl-2-oxoethyl)-3- (methanesulfonyl hydrazinocarbonyl) pyridinium bromide (compound 68);
N, N'-Bis [3-carbonyl-1-(2-thien-2'-yl-2-oxoethyl) pyridinium] hydrazine dibromide (compound 69);
1- (2-Ethoxy-2-oxoethyl)-3- (2- (benzoyloxy) ethylaminocarbonyl) pyridinium bromide (compound 70);
1- (2- (2,4-Dichlorophenyl)-2-oxoethyl)-3- (2-(benzoyloxy) ethylamino- carbonyl) pyridinium bromide (compound 71);
1- (2-Thien-2'-yl-2-oxoethyl)-3- (2- (2-pyridyl) hydrazinocarbonyl) pyridinium bromide (compound 72);
1- (2-Phenyl-2-oxoethyl)-3- (2- (2-pyridyl) hydrazinocarbonyl) pyridinium bromide (compound 73);
1- (2-Phenyl-2-oxoethyl)-3- (hydrazinocarbonyl) pyridinium bromide (compound 74);
1- (2- Phenyl-2-oxoethyl)-3- (methanesulfonyl hydrazinocarbonyl) pyridinium bromide (compound 75);
1- (2-Ethoxy-2-oxoethyl)-3- (methanesulfonyl hydrazinocarbonyl) pyridinium bromide (compound 76);
1- (2-Phenyl-2-oxoethyl)-3- (phenylsulfonylhydrazino carbonyl) pyridinium bromide (compound 77);
1- (2-Phenyl-2-oxoethyl)-2-chloro-3- (phenylsulfonylhydrazino carbonyl) pyridinium bromide (compound 78);
1- (2- Phenyl-2-oxoethyl)-3- (2- (methoxy) carbonyl) ethyloxy carbonyl pyridinium bromide (compound 79);
1- (2-Ethoxy-2-oxoethyl)-3- (2- (benzoyloxy) ethyloxy carbonyl) pyridinium bromide (compound 80);
1-(2-Thien-2'-yl-2-oxoethyl)-4-(2-(benzoyloxy) ethylaminocarbonyl) pyridinium bromide (compound 81);
1- (2-thien-2'-yl-2-oxoethyl)-3- [(3-phenyl methyl)pyrazol-5-yl] pyridinium bromide (compound 82);
1-(2-thien-2'-yl-2-oxoethyl)-3-[(3-phenyl methyl) oxazol-5-yl pyridinium bromide (compound 83);
1-(2-thien-2'-yl-2-oxoethyl)-3-[3-{1-(2-thien-2'-yl)-2-oxoethyl pyridinium-4 thio} methyl-pyrazol-5-yl] pyridinium dibromide. (compound 84);
1-(2-thien-2'-yl-2-oxoethyl)-3-[3- f1-(3,5-dimethylpyrazol-1-yl) methyl} pyrazol-5-yl] pyridinium bromide. (compound 85);
1- (2-thien-2'-yl-2-oxoethyl)-3-[3-phenylmethyl}-1-{2-pyridyl}-pyrazol-5-yl]-pyridinium bromide. (compound 86);
1- (2-thien-2'-yl-2-oxoethyl)-3-[3(3,5-dimethylpyrazol-1-yl)methyl-1-pyridyl} pyrazol-5-yl] pyridinium bromide. (compound 87);
1- [2- (cyclopropylamino)- 2-oxoethyl] 3-[3-(3,5-dimethylpyrazol-1-yl)methyl}-pyrazol-5-yl]-pyridinium bromide. (compound 88);
1-{2-(4-nitro-2-thienyl)-2-oxoethyl}-3-[3{(3,5-dimethylpyrazol-1-yl)methyl} pyrazol-5-yl]-pyridinium bromide. (compound 89);
1- (2-cyclopropylamino-2-oxoethyl)-3[(3-phenylmethyl) pyrazol-5-yl] pyridinium chloride. (compound 90);
3,5-bis-[1-(2-thien-2'-yl-2-oxoethyl)-pyridinium-3-yl]-pyrazole dibromide. (compound 91);
1-(2-thien-2'-yl-2-oxoethyl)-3-1 (1-phenyl-3-phenylmethyl)pyrazol-5-yl]pyridinium chloride. (compound 92);
1-(2-(5'-methyl-2-thienyl)-2-oxoethyl)-3-[(3-phenylmethyl) pyrazol-5-yl] pyridinium chloride. (compound 93);
1- (2-thien-2'-yl-2-oxoethyl) 3- [1-phenyl,3-{(3,5-dimethyl pyrazol-1-yl) methyl)} pyrazol-5-yl]-pyridinium chloride. (compound 94);
1- (2-phenyl-2-oxoethyl)-3-[(3-phenylmethyl)pyrazol-5-yl]-pyridinium bromide. (compound 95);
1- (2-cyclopropylamino- 2-oxoethyl) 3-[(1-phenyl-3-phenylmethyl)pyrazol-5 yl]-pyridinium chloride (compound 96);
1- (2- (4-benzyl-l-piperidinyl)-2-oxoethyl) 3-[(3-phenoxymethyl)pyrazol-5-yl] pyridinium bromide (compound 97);
1 (2-phenyl-2-oxoethyl)-3-[(3-(3, 5-dimethylpyrazol-1-yl) methyl) pyrazol-5 yl]-pyridinium chloride (compound 98);
1-(2-(5-methyl-2-thienyl)-2-oxoethyl)-3-[(3,-(3, 5-dimethylpyrazol-1-yl) methyl). pyrazol-5-yl] pyridinium chloride (compound 99);
1- (2-phenyl-2-oxoethyl)-3-[(1-phenyl-3-phenylmethyl) pyrazol-5-yl] pyridinium chloride (compound 100);
1- (2- (5-methyl-2-thienyl)-2-oxoethyl)-3-[(3(2-cyclohexyl ethyl) pyrazol-5-yl] pyridinium chloride (compound 101);
1- (2-cyclopropylamino-2-oxoethyl)-3-[(3-(2-cyclohexylethyl) pyrazol-5-yl] pyridinium chloride (compound 102);
1- (2-phenyl-2-oxoethyl)-3-[(3-(2-cyclohexylethyl) pyrazol-5-yl] pyridinium chloride (compound 103);
1- (2-cyclopropylamino-2-oxoethyl)-3-[(1-cyclohexyl-3-phenylmethyl) pyrazol 5-yl] pyridinium chloride (compound 104);
1- (2-thien-2'-yl-2-oxoethyl)-3-[(3-phenoxymethyl) pyrazol-5-yl] pyridinium chloride (compound 105);
1- [2- (1-adamantylamino)-2-oxoethyl]-3-[(3-phenylmethyl) pyrazol-5-yl] pyridinium chloride (compound 106);
1- (2-phenyl-2-oxoethyl)-3-[{3-(3,5-dimethylpyrazol-1-yl)methyl)}1-phenyl-pyrazol-5-yl] pyridinium bromide (compound 107);
1-(2-(4-nitor-2-thienyl)-2-oxoethyl)-3-[(1-cyclohexyl-3-(3,5-dimethylpyrazol-1 yl)-methyl) pyrazol-5-yl] pyridinium bromide (compound 108);
1- (2- (4-nitro-2-thienyl)-2-oxoethyl)-3[(3-(2-cyclohexylethyl) pyrazol-5-yl] pyridinium bromide (compound 109);
1-(2-thien-2'-yl-2-oxoethyl)-3-[(1-phenyl-3-phenoxymethyl) pyrazol-5-yl] pyridinium chloride (compound 110);
1- (2- (4-nitro-2-thienyl)-2-oxoethyl)-3-[(1-phenyl-3-phenylmethyl) pyrazol-5 yl] pyridinium bromide (compound 111);
pyrazolel- (2-cyclopropylamino-2-oxoethyl)-3-[(3-phenoxymethyl) pyrazol-5-yl] pyridinium chloride (compound 112);
1- (2-cyclopropylamino-2-oxoethyl)-3-[(1-cyclohexyl-3-(3,5-dimethyl pyrazole)-1-yl)-5-yl] pyridinium chloride (compound 113);
1- (2- (5-chloro-2-thienyl)-2-oxoethyl)-3- [ (3-phenoxymethyl) pyrazol-5-yl] pyridinium bromide (compound 114);
1-(2-phenyl-2-oxoethyl)-3-[(1-phenyl-3-phenoxyrnethyl) pyrazol-5-yl] pyridinium chloride (compound 115);
1- (2-thien-2'-yl-2-oxoethyl)-3- [ (1-cyclohexyl-3- (3, 5-dimethylpyrazol-1-yl-methyl) pyrazol-5-yl] pyridinium chloride (compound 116);
1- (2-cyclopropylamino-2-oxoethyl)-3- [(1-phenyl-3-phenoxymethyl) pyrazol-5 yl] pyridinium bromide (compound 117);
1- (2-thien-2'-yl-2-oxoethyl)-3- [(1-phenyl-3- (2-cyclohexylethyl)pyrazol-5-yl] pyridinium bromide (compound 118);
1-(2-thien-2'-yl-2-oxoethyl)-3-[( 1-cyclohexyl-3-phenoxymethyl)pyrazol-5-yl] pyridinium chloride (compound 119);
3- [ (3-phenylmethyl) pyrazol-5-yl] pyridine hydrochloride (compound 120);
3- [ (3-phenoxymethyl) pyrazol-5-yl] pyridine hydrochloride (compound 121);
3- [ (3, 5-dimethylpyrazol-1-yl-methyl) pyrazol-5-yl] pyridine (compound 122) ;
3- [3- (2-cyclohexyl-ethyl)-pyrazol-5-yl] pyridine (compound 123);
1- (2-napthyl-2-oxo ethyl)-3 [ (3-phenoxymethyl) pyrazol-5-yl] pyridinium bromide (compound 124);
1- (phenylmethyl)-3 [ (3-phenyl methyl)pyrazol-5-yl] pyridinium chloride (compound 125);
1-(2-thien-2'-yl-2-oxo ethyl)-3[ (3 (-1-naphthyl) pyrazol-5-yl] pyridinium chloride (compound 126);
1- (2-phenyl-2oxoethyl)-3 [3 (thienyl-2-yl-methyl)pyrazol-5-yl] pyridinium chloride (compound 127);
1- (2- (5-methyl-2-thienyl)-2-oxoethyl)-3- [3 (2-phenyl ethyl) pyrazol-5 yl] pyridinium chloride (compound 128);
1- (2- (5-methyl 2-thienyl)-2-oxo ethyl)-3- [3- (3-phenoxy propyl) pyrazol-5 yl] pyridinium chloride (compound 129);
1- (isopropyl)-3 [ (3-phenylmethyl) pyrazol-5-yl] pyridinium bromide (compound 130);
1- (2- (5-methyl-2-thienyl)-2-oxoethyl)-3- [ (3-thiophenylmethyl) pyrazol-5 yl] pyridinium chloride (compound 131);
1- (2-thien-2'-yl-2-oxoethyl)-3 [ (3- (N-methyl-indole-3-yl methyl) pyrazol-5 yl] pyridinium chloride (compound 132);
1- (2-napthyl-2-oxo-ethyl)-3 [ (3-methyl) pyrazol-5-yl] pyridinium bromide (compound 133);
1- (2- (1, 4 benzodioxane-6-yl-amino-2-oxoethyl)-3 [ (3-phenylmethyl) pyrazol-5-yl] pyridinium chloride (compound 134);
1- (2-thien-2'-yl-2-oxo. ethyl)-3[ (3-phenyl) pyrazol-5-yl]-5 bromo pyridinium chloride (compound 135);
1- (2-thien-2'-yl)-2-oxoethyl)-3 [ (3-phenyl) pyrazol-5-yl] quinolinium chloride (compound 136) and
3- [ (3-phenyl) pyrazol-5-yl)] quinoline (compound 137).

In a preferred embodiment the present invention relates to pharmaceutical combination or compositions which comprises compound no. 27 and/or 68 or a pharmaceutically acceptable salts thereof-and at least one therapeutic agent selected from the group consisting of: an antihypertensive agent; an antidiabetic agent; a hypolipidemic agent; an antiplatelet agent; an antiobesity agent; an antithrombotic agent; an agent for diabetic vascular complications; and an agent for treatment of heart failure; or a pharmaceutically acceptable salts thereof. More preferably combination or composition comprises in combination compound no. 27 or a pharmaceutically acceptable salts thereof and at least one therapeutic agent selected from metformin, glimepiride, pioglitazone, sitagliptin, vildagliptin, amlodipine, felodipine, diltiazem, lercanidipine, ramipril, enalapril, lisinopril, perindopril, atorvastatin, simvastatin, rosuvastatin, pravastatin, fluvastatin, aliskiren, nicorandil, indapamide, clopidogrel, prasugrel, aspirin , hydrochlorothiazide, rivaroxaban, indapamide, furosemide or its pharmaceutically acceptable salts thereof.

The antihypertensive agent, as mentioned herein, includes but not limited to an angiotensin converting enzyme (ACE) inhibitor, a renin inhibitor, a beta adrenergic receptor blocker, an alpha adrenergic receptor blocker, a calcium channel blocker, a potassium channel activator, an aldosterone synthase inhibitor, a neutral endopeptidase (NEP) inhibitor, a dual angiotensin converting enzyme/neutral endopeptidase (ACE/NEP) inhibitor, an endothelin receptor antagonist, a dual angiotensin and endothelin receptor antagonist (DARA), a diuretic or a pharmaceutically acceptable salt thereof.

The interruption of the enzymatic degradation of angiotensin I to angiotensin II with so-called ACE-inhibitors is a successful variant for the regulation of blood pressure and also a therapeutic method for the treatment of congestive heart failure.

Numerous ACE inhibitors have been synthesized. Most of these compounds can be classified into three groups based on their chemical structure: (1) sulfhydryl-(also called mercapto-) containing ACE inhibitors, including captopril and agents that are structurally related to captopril, such as fentiapril, pivalopril, zofenopril and alacepril; (2) dicarboxyl-containing ACE inhibitors, including enalapril and agents that are structurally related to enalapril, such as lisinopril, benazepril, quinapril, moexipril, ramipril, spirapril, perindopril, indolapril, pentopril, indalapril and cilazapril; and (3) phosphorus-containing ACE inhibitors, structurally related to fosinopril. Representative ACE inhibitors are captopril, cilazapril, delapril, enalapril, enalaprilat, fentiapril, fosinopril, indolapril, libenzapril, rentiapril, zabicipril, moveltipril, spiraprilat, lisinopril, perindopril, pivopril, quinapril, ramipril, spirapril, trandolapril, and zofenopril; particularly preferred are captopril, enalapril, perindopril, fosinopril, lisinopril, quinapril, ramipril, and trandolapril; or in each case, a pharmaceutically acceptable salt thereof. Preferred ACE inhibitors are those agents that have been marketed, most preferred are benazepril, ramipril, perindopril, lisinopril, enalapril or a pharmaceutically acceptable salt thereof.

Renin released from the kidney cleaves angiotensinogen in the circulation to form the decapeptide angiotensin I. This is in turn cleaved by angiotensin converting enzyme in the lungs, kidneys and other organs to form the octapeptide angiotensin II, which increases blood pressure both directly by arterial vasoconstriction and indirectly by liberating from the adrenal glands the sodium-ion-retaining hormone aldosterone, accompanied by an increase in extracellular fluid volume. Inhibitors of the enzymatic activity of renin bring about a reduction in the formation of angiotensin I. As a result a smaller amount of angiotensin II is produced. The reduced concentration of that active peptide hormone is the direct cause of e.g. the antihypertensive effect of renin inhibitors. Accordingly, renin inhibitors or salts thereof can be employed e.g. as antihypertensives or for treating congestive heart failure.

The class of renin inhibitor comprises compounds having differing structural features. For example, mention may be made of compounds which are selected from the group comprising of Aliskiren, ditekiren, terlakiren, zankiren, RO 66-1132, RO-66-1168 or in each case, a pharmaceutically acceptable salt thereof. Especially preferred is aliskiren or a pharmaceutically acceptable salt, especially the hemi-fumarate, thereof.

The beta adrenergic receptor blocker in said combination comprises selective or nonselective beta blocker, preferably is a representative selected from the group comprising of a selective beta.1-blockers, such as atenolol, bisoprolol, metoprolol, esmolol, celiprolol, betaxololor taliprolol or a non-selective .beta.-blocker, such as oxprenolol, pindolol, propranolol, timolol, bupranolol, penbutolol, mepindolol, carteolol or nadolol, or a .beta.-blockers possessing also .alpha.-blocking activity such as carvedilol or labetalol, or in each case, a pharmaceutically acceptable salt thereof.

The alpha-1 adrenergic receptor blocker in said combination preferably is a representative selected from the group consisting of doxazosin, prazosin, terazosin or in each case, a pharmaceutically acceptable salt thereof.

The class of calcium channel blocker (CCBs) essentially comprises dihydropyridines (DHPs) and non-DHPs such as diltiazem-type and verapamil-type CCBs. A CCB useful in said combination is preferably a DHP representative selected from the group comprising of amlodipine, lercanidipine, felodipine, isradipine, lacidipine, nicardipine, nifedipine, niguldipine, niludipine, nimodipine, nisoldipine, nitrendipine, and nivaldipine, and is preferably a non-DHP representative selected from the group comprising of flunarizine, prenylamine, diltiazem, fendiline, gallopamil, mibefradil, anipamil, tiapamil and verapamil, or in each case, a pharmaceutically acceptable salt thereof.

Preferred CCBs comprise amlodipine, lercanidipine, diltiazem, isradipine, nicardipine, nifedipine, nimodipine, nisoldipine, nitrendipine, and verapamil, or pharmaceutically acceptable salt thereof. An especially preferred DHP is amlodipine or a pharmaceutically acceptable salt, especially the besylate. An especially preferred representative of non-DHPs is diltiazem or verapamil or a pharmaceutically acceptable salt, especially the hydrochloride.

The preferred potassium channel activators include without limitation nicorandil including its pharmaceutically acceptable salts.

Aldosterone synthase is an enzyme that converts corticosterone to aldosterone by hydroxylating corticosterone to form 18-OH-corticosterone and 18-OH-corticosterone to aldosterone. The term "aldosterone synthase inhibitor" denotes a compound that directly or indirectly reduces or stops the synthesis or activity of aldosterone. The class of aldosterone synthase inhibitors is known to be applied for the treatment of hypertension and primary aldosteronism comprises both steroidal and non-steroidal aldosterone synthase inhibitors, the later being most preferred.

Preference is given to commercially available aldosterone synthase inhibitors or those aldosterone synthase inhibitors that have been approved by the health authorities.

The terms "aldosterone antagonist" and "aldosterone receptor antagonist" denote a compound capable of binding to an aldosterone receptor, as a competitive inhibitor of the action of aldosterone itself at the receptor site, so as to modulate the receptor-mediated activity of aldosterone. Such compounds are also contemplated in the present invention.

Certain compounds, for example 11 β-Hydroxy androst-4-en-3-one 17-spirolactone, act as both an aldosterone synthase inhibitor and as an aldosterone receptor antagonist. Such compounds are also contemplated in the present invention.

The natriuretic peptides constitute a family of peptides that include the atrial (ANP), brain-derived (BNP) and C-type natriuretic (CNP) peptides. The natriuretic peptides affect vasodilation, natriuresis, diuresis, decreased aldosterone release, decreased cell growth, and inhibition of the sympathetic nervous system and the renin-angiotensin-aldosterone system indicating their involvement in the regulation of blood pressure and of sodium and water balance. Neutral endopeptidase 24.11 (NEP) inhibitors impede degradation of natriuretic peptides and elicit pharmacological actions potentially beneficial in the management of several cardiovascular disorders. NEP inhibitor useful in the said combination is an agent selected from the group represented by candoxatril, sinorphan, SCH 34826 and SCH 42495 or in each case, a pharmaceutically acceptable salt thereof.

Compounds having inhibitory effects on both angiotensin converting enzyme and neutral endopetidase, so-called dual ACE/NEP inhibitors, can be used for the treatment of cardiovascular pathologies. A preferred dual angiotensin converting enzyme/neutral endopetidase (ACE/NEP) inhibitor is, for example, omapatrilat, fasidotril, fasidotrilat, Z13752A or a pharmaceutically acceptable salt thereof.

Compounds having dual angiotensin and endothelin receptor antagonist (DARA) action can be used for the treatment of cardiovascular pathologies. A preferred compound is PS433540 and compound disclosed in international publication WO/2007/100295

Endothelin (ET) is a highly potent vasoconstrictor peptide synthesized and released by the vascular endothelium. Endothelin (ET) exists in three isoforms (ET-1, ET-2 and ET-3). (ET shall mean any or all of the isoforms of ET). Elevated levels of ET have been reported in plasma from patients with essential hypertension. Endothelin receptor antagonists can be used to inhibit the vasoconstrictive effects induced by ET.

The preferred endothelin antagonists are, for example, bosentan, enrasentan, atrasentan, especially atrasentan hydrochloride, darusentan, BMS 193884, sitaxsentan, especially sitaxsentan sodium, YM 598, S 0139, J 104132, furthermore, tezosentan, or in each case, a pharmaceutically acceptable salt thereof.

The diuretic in this composition is selected from the group comprising of hydrochlorothiazide (HCTZ), furosemide, altizide, trichlormethazide, triflumethazide, bemetizide, cyclothiazide, methylchlothiazide, azosemide, chlorothiazide, butizide, bendroflumethazide, cyclopenthiazide, benzclortriazide, polythiazide, hydroflumethazide, benzthiazide, ethiazide, penflutazide, chlorthalidone, butazolimide, spironolactone, amiloride, indapamide and triamterene. Preferred diuretic for incorporation in this invention are hydrochlorothiazide, indapamide, trichlormethazide, furosemide, chlorthalidone, and altizide, especially hydrochlorothiazide or in each case, a pharmaceutically acceptable salt thereof.

The hypolipidemic agent or lipid-lowering agent which may be employed in combination with the compounds of formula (I) and/or (II) include without limitation, a MTP inhibitor, a HMG CoA reductase inhibitor, a squalene synthetase inhibitor, a fibric acid derivative, an ACAT inhibitor, a lipoxygenase inhibitor, a cholesterol absorption inhibitor, an ileal Na+/bile acid cotransporter inhibitor, an upregulator of LDL receptor activity, a cholesteryl ester transfer protein(CETP) inhibitor, a bile acid sequestrant, and/or nicotinic acid and derivatives or a pharmaceutically acceptable salt thereof.

The HMG-CoA reductase inhibitor used in the present invention means any drug that inhibits 3-hydroxy-3-methylglutaryl-coenzyme A reductase (HMG-CoA reductase), which is an enzyme in rate-determining step of cholesterol biosynthesis, and includes without limitation atorvastatin, pravastatin, cerivastatin, simvastatin, lovastatin, fluvastatin, mevastatin, itavastatin, rosuvastatin, pitavastatin and ZD4522 or in each case, a pharmaceutically acceptable salt thereof;. atorvastatin, pravastatin, fluvastatin, and simvastatin are preferred.

The squalene synthetase inhibitor suitable for use herein include, but are not limited to, ER-27856, ER-28448, RPR 107393 or in each case, a pharmaceutically acceptable salt thereof, as well as other known squalene synthetase inhibitors,.

Other hypolipidemic agents suitable for use herein include, but are not limited to, fibric acid derivatives, such as fenofibrate, gemfibrozil, clofibrate, bezafibrate, ciprofibrate, clinofibrate, probucol, or in each case, a pharmaceutically acceptable salt thereof and related compounds, probucol and gemfibrozil being preferred, bile acid sequestrants such as cholestyramine, colestipol and DEAE-Sephadex, as well as lipostabil, Eisai E-5050, imanixil, tetrahydrolipstatin (THL), istigmastanylphosphorylcholine, aminocyclodextrin, AJ-814 (azulene derivative), melinamide, Sandoz58-035, CL-277,082 and CL-283,546 (disubstituted urea derivatives), nicotinic acid, acipimox, acifran, neomycin, p-aminosalicylic acid, aspirin, poly(diallylmethylamine) derivatives, quaternary amine poly (diallyldimethylammonium chloride) and ionenes. The hypolipidemic agent may also be an ileal Na +/bile acid cotransporter inhibitor such as S-8921.

The acyl coenzyme A-cholesterol acyl transferase (ACAT) inhibitor useful in the present invention includes, but are not limited to, avasimibe, eflucimibe, KY505, SMP 797, or in each case, a pharmaceutically acceptable salt thereof.

The cholesterol absorption inhibitor useful in the present invention includes, but not limited to, stanol esters, beta-sitosterol; sterol glycosides such as tiqueside; and azetidinones such as ezetimibe or pharmaceutically acceptable salt thereof; A preferred cholesterol absorption inhibitor is ezetimibe or its pharmaceutically acceptable salts.

The CETP inhibitor useful in the present invention includes, but not limited to, JTT 705, torcetrapib, CP 532,632, BAY63-2149, SC 591, SC 795, or in each case, a pharmaceutically acceptable salt thereof.

The antidiabetic agent, as mentioned herein, includes but not limited to a PPARγ agonist, a biguanide, a protein tyrosine phosphatase-1B (PTP-1B) inhibitor, a sulfonylurea, a meglitinide, an alpha glucoside hydrolase inhibitor, a PPARα agonist, a PPARδ agonist or antagonist, an alpha-amylase inhibitor, a fatty acid oxidation inhibitor, an A2 antagonist, a dipeptidyl peptidase IV (DP4) inhibitor, an aP2 inhibitor, a SGLT2 inhibitor, a glycogen phosphorylase inhibitor, a glucagon-like peptide-1 (GLP-1), an insulin or insulin mimetic, a PPAR.alpha./gamma dual agonist, an 11β-HSD 1 (11β-hydroxy-steroid dehydrogenase 1) inhibitor, other insulin sensitizing drug, a glucokinase activator, a VPAC2 receptor agonist or a pharmaceutically acceptable salt thereof.

The antidiabetic agent may be an oral antihyperglycemic agent preferably a biguanide such as metformin or phenformin or salts thereof, preferably metformin HC1.

The other antidiabetic agent may be a sulfonylurea such as glyburide (also known as glibenclamide), glimepiride, glipizide, gliclazide or chlorpropamide or in each case, a pharmaceutically acceptable salt thereof, other known sulfonylurea or other antihyperglycemic agent which act on the ATP-dependent channel of the β-cells, with glyburide and glipizide being preferred.

The oral antidiabetic agent may also be an alpha glucoside hydrolase inhibitor such as acarbose, adiposine, camiglibose, emiglitate, miglitol, voglibose, pradimicin-Q, salbostatin, CKD-711, MDL-25,637, MDL-73,945, MOR-14 or a pharmaceutically acceptable salt thereof.

The PPARγ agonist may be selected from thiazolidinedione oral anti-diabetic agents or other insulin sensitizers such as rosiglitazone, pioglitazone, MCC-555, GL-262570, englitazone, darglitazone, isaglitazone, JTT-501, L-895645, R-119702, NN-2344, balaglitazone or YM-440 or in each case, a pharmaceutically acceptable salt thereof, preferably rosiglitazone and pioglitazone.

The compounds of formula (I) and/or (II) may also be employed in combination with an antihyperglycemic agent such as insulin or insulin mimetic like biota, LP-100, novarapid, insulin detemir, insulin lispro, insulin glargine, insulin zinc suspension (lente and ultralente), Lys-Pro insulin or glucagon-like peptide-1 (GLP-1) such as GLP-1(1-36) amide, GLP-1(7-36) amide, GLP-1(7-37) (as disclosed in U.S. Pat. No. 5,614,492 the disclosure of which is incorporated herein by reference), as well as AC2993 and LY-315902, which may be administered via injection, intranasal, inhalation or by transdermal or buccal devices.

The other antidiabetic agent may also be a PPAR α/γ dual agonist such as CLX-0940, GW-1536, GW1929, GW-2433, KRP-297, L-796449, LR-90, MK-0767, SB 219994, muraglitazar, AZ-242/tesaglitazar, GW-409544, or in each case, a pharmaceutically acceptable salt thereof.

The antidiabetic agent may be a SGLT2 inhibitor such as Sergliflozin, Dapagliflozin or or pharmaceutically acceptable salt thereof. Preferred will be Sergliflozin. The antidiabetic agent may also be an aP2 inhibitor. PTP1-B inhibitor can be selected from the compounds disclosed in international publication WO/2007/32028.

The antidiabetic agent may be a DPP4 (Dipeptidyl peptidase IV) inhibitor such as, vildagliptin, sitagliptin, saxagliptin, alogliptin or in each case, a pharmaceutically acceptable salt thereof. The most preferred is vildagliptin.

The meglitinide which may be employed in combination with the compound of formula (I) of the invention includes but not limited to repaglinide, nateglinide or KAD1229 or its pharmaceutically acceptable salt thereof, with repaglinide being preferred.

The alpha-amylase inhibitor may be employed in combination include but not limited to tendamistat, trestatin, A1-3688, and the like. The A2 antagonist can be selected from midaglizole, isaglidole, deriglidole, idazoxan, earoxan, and fluparoxan. The fatty acid oxidation inhibitor, which may be employed in combination, is clomoxir, etomoxir, and the like.

The antiobesity agent, as mentioned herein, include but not limited to a 5HT (serotonin) transporter inhibitor, a NE (norepinephrine) transporter inhibitor, a CB-1 (cannabinoind-1 receptor) antagonist/inverse agonist, a ghrelin antibody, a ghrelin antagonist, a H3 (histamine H3) antagonist/inverse agonist, a NPY1 (neuropeptide Y Y1) antagonist, a NPY2 (neuropeptide Y Y2) agonist, a NPY5 (neuropeptide Y Y5) antagonist, leptin or its derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a β3 (beta adrenergic receptor 3) agonist, a DGAT1 (diacylglycerol acyltransferase 1) inhibitor, a DGAT2 (diacylglycerol acyltransferase 2) inhibitor, a FAS (fatty acid synthase) inhibitor, a PDE (phosphodiesterase) inhibitor, a thyroid hormone β agonist, an UCP-1 (uncoupling protein 1), 2,.or 3 activator, an acyl-estrogen, a glucocorticoid antagonist, a SCD-1 (stearoyl-CoA desaturase-1) inhibitor, a lipase inhibitor, a fatty acid transporter inhibitor, a dicarboxylate transporter inhibitor, or pharmaceutically acceptable salts.

The serotonin (5HT) transport inhibitor useful in this invention includes but not limited to, paroxetine, fluoxetine, fenfluramine, fluvoxamine, sertraline, imipramine or in each case, a pharmaceutically acceptable salt thereof.

The norepinephrine (NE) transport inhibitor useful in this invention includes but not limited to, GW 320659, despiramine, talsupram, nomifensine or in each case, a pharmaceutically acceptable salt thereof.

The cannabinoid receptor 1 (CB-1) antagonist/inverse agonist useful in the present invention includes but not limited to rimonabant, taranabant, rosonabant, CP-945598, SR-147778, BAY 65-2520, SLV 319 or in each case, a pharmaceutically acceptable salt thereof.

The neuropeptide Y1 (NPY1) antagonists useful in the present invention includes but not limited to BIBP3226, J-115814, BIBO 3304, LY-357897, CP-671906, GI-264879A or in each case, a pharmaceutically acceptable salt thereof.

The neuropeptide Y2 (NPY2) agonist useful in the present invention includes but not limited to, peptide YY (PYY), and PYY3-36, peptide YY analogs, PYY agonists.

The neuropeptide Y5 (NPY5) antagonist useful in the present invention includes but not limited to GW-569180A, GW-594884A, GW-587081X, GW-548118X; FR 235,208; FR226928, FR 240662, FR252384; 1229U91, GI-264879A, CGP71683A, LY-377897, LY366377, PD-160170, SR-120562A, SR-120819A, JCF-104, H409/22 or in each case, a pharmaceutically acceptable salt thereof.

The beta.3 adrenergic receptor (beta.3) agonist useful in the present invention includes but not limited to AD9677/TAK677, CL-316,243, SB 418790, BRL-37344, L-796568, BMS-196085, BRL-35135A, CGP12177A, BTA-243, GW 427353, Trecadrine, Zeneca D7114, SR 59119A or in each case, a pharmaceutically acceptable salt thereof

The thyroid hormone beta agonist useful in the present invention includes but not limited to MB07811, KB-2611 or its pharmaceutically acceptable salt thereof.

The antiplatelet agent which may be employed in combination with compounds of formula (I) and/or (II) of the invention includes without limitation aspirin, clopidogrel, ticlopidine, dipyridamole, prasugrel, abciximab, tirofiban, eptifibatide, anagrelide, ifetroban or in each case, a pharmaceutically acceptable salt thereof, with clopidogrel, prasugrel, and aspirin being preferred.

The anti-thrombotic agent which may be employed in combination with compounds of formula (I) and/or (II) of the invention includes without limitation melagatran and ximelagatran (Exanta® Astra Zeneca), warfarin and Factor Xa inhibitors such as rivaroxaban, razaxaban or in each case, a pharmaceutically acceptable salt thereof.

An agent useful for diabetic vascular complications in present invention includes without limitation aldose reductase inhibitor, AGE inhibitor or AGE breaker. Aldose reductase inhibitor, among those suitable for the treatment of diabetic complications, represent those which decrease intracellular sorbitols by inhibiting aldose reductases, and said sorbitols accumulate excessively by enhancement of a course of polyol metabolism which is induced by continuous hyperglycemia shown in tissues developing diabetic complication. They include, for example, epalrestat, tolrestat, fidarestat zenerestat or its pharmaceutically acceptable salt thereof. Advanced glycation end products (AGE) inhibitor, among those suitable for the treatment of diabetic complications, represent those which alleviate cell disorders by inhibiting production of advanced glycation endproducts which are increased by continuous hyperglycemia in a diabetic state. NNC-39-0028 and OPB-9195 are examples thereof. AGE breaker suitable for the treatment of diabetic complications, represent those which are capable of reversing the formation of advanced glycosylation end product cross-links responsible for the complications of aging and diabetes. Alagebrium is non limiting example of AGE breaker.

The digitalis glycoside, which can be used in present invention includes without limitation digitoxin, gitoxin, gitalin, digoxin, F-gitonin, digitonin,. Examples of phosphodiesterase inhibitors are amrinone, milrinone, enoximone and bucladesine or in each case, a pharmaceutically acceptable salt thereof.

The compounds to be combined can be present as a base or the most stable and potent or clinically proven pharmaceutically acceptable salts. If these compounds have, for example, at least one basic center, they can form acid addition salts. Corresponding acid addition salts can also be formed having, if desired, an additionally present basic center. The compounds having at least one acid group (for example COOH) can also form salts with bases. Corresponding internal salts may furthermore be formed, if a compound comprises, e.g., both a carboxy and an amino group.

The corresponding active ingredient or a pharmaceutically acceptable salt thereof may also be used in crystalline form, amorphous form, hydrate or include other solvates.

In a preliminary study (Phase-I) using compound of formula I and/or II, when compound was administered in conjunction with amlodipine, doxasozin, metformin, aspirin, simvastatin, ezetimibe and latanoprost, it was found to be safe and did not result in to any adverse events. This indicates that the compound is safe for coadministration with multiple class of compounds.

It has been found that, a combination of compound of formula (I) and/or (II) and at least one therapeutic agent selected from the group consisting of: an antihypertensive agent; an antidiabetics agent; a hypolipidemic agent; an antiplatelet agent; an antiobesity agent; an antithrombotic agent; an agent for diabetic vascular complications; and an agent for treatment of heart failure; or a pharmaceutically acceptable salt thereof, achieves greater therapeutic response.

### Definitions:

The use of the terms "a" and "an" and "the" and similar referents in the context of describing the invention (especially in the context of the following claims) are to be construed to cover both the singular and the plural, unless otherwise indicated herein or clearly contradicted by context.

The terms "comprising", "having", "including", and "containing" are to be construed as open-ended terms (i.e., meaning "including, but not limited to") unless otherwise noted.

The term "treatment", "treat", "treating", refer to both, prophylactic or preventative treatment as well as curative or disease modifying treatment, including treatment of patients at risk of contracting the disease or suspected to have contracted the disease as well as patients who are ill or have been diagnosed as suffering from a disease or medical condition.

The term "therapeutically effective amount" refers to that amount of a compound of the invention that is sufficient to effect treatment, as defined above, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

The term "at least one therapeutic agent" shall mean that in addition to compound of formula (I) and/or (II) one or more, for example two, furthermore three, active ingredients as specified according to the present invention can be combined.

The active agents of the invention may be present in the same pharmaceutical compositions or in separate pharmaceutical compositions. Thus the active ingredients may be administered at the same time (e.g. simultaneously) or at different times (e.g. sequentially) and over different periods of time, which may be separate from one another or overlapping. The unit dose form may also be a fixed combination of desired active ingredients.

The pharmaceutical combination of present invention can be administered by oral, parenteral (e.g., intramuscular, intraperitoneal, intravenous, ICV, intracistemal injection or infusion, subcutaneous injection, or implant), by inhalation spray, nasal, vaginal, rectal, sublingual, or topical routes of administration and may be formulated, alone or together with suitable conventional pharmaceutically acceptable carriers, adjuvants and vehicles appropriate for each route of administration. The preferred route is oral.

Typical oral formulations include tablets, capsules, syrups, elixirs and suspensions. Typical injectable formulations include solutions and suspensions.

The pharmaceutical compositions intended for oral use may be prepared according to any suitable method known to person skilled in art for the manufacture of pharmaceutical compositions. For example tablets may contain the active ingredient in admixture with pharmaceutically acceptable excipients which are suitable for the manufacture of tablets. These excipients without limitation include, diluents, such as calcium carbonate, sodium carbonate, lactose, calcium phosphate or sodium phosphate; granulating and disintegrating agents, such as corn starch, crosspovidone, or alginic acid; binders, for example starch, gelatin or acacia, and lubricating agents, for example magnesium stearate, stearic acid or talc. The tablets may be uncoated or as required coated by known methods to delay disintegration and absorption in the gastrointestinal tract and thereby to provide a delayed or sustained action over a period of time. Pharmaceutical compositions for oral use may also be prepared as hard gelatin capsules or as soft gelatin capsules using suitable pharmaceutically acceptable excipients by any known method in art..

Aqueous suspensions can be prepared containing the active materials in admixture with excipients suitable for the manufacture of aqueous suspensions. Such excipients without limitation include suspending agents, such as sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. One or more preservatives, for example ethyl, or n-propyl, p-hydroxybenzoate, one or more coloring agents, one or more flavoring agents, and one or more sweetening agents, such as sucrose or saccharin may be added to the aqueous suspensions in case if required.

Oily suspensions may be formulated by known methods using acceptable excipients such as suspending agents like vegetable oil such as olive oil, coconut oil, or mineral oil such as liquid paraffin; thickening agents, like beeswax, hard paraffin or cetyl alcohol; Sweeteners; and flavoring agents. These compositions may be preserved by the addition of an anti-oxidant such as ascorbic acid.

The pharmaceutical compositions of the invention may also be in the form of oil-in-water emulsions. The oily phase may be a vegetable oil, a mineral oil, or mixtures of these. Suitable emulsifying agents may be employed for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate.

Parenteral formulations can be administered intravenously, intramuscularly, intraperitoneally, intranasally, intradermally or subcutaneously. suitable parenteral formulation can be prepared using pharmaceutical excipients with known method in the art.

The compounds of the present invention may also be presented in the form of suppositories for rectal administration of the drug based on suitability. These compositions can be prepared using a suitable non-irritating excipient which is solid at ordinary temperatures but liquid at the rectal temperature and will therefore melt in the rectum to release the drug. Such examples are cocoa butter and polyethylene glycols.

Suitable formulations for topical application, e.g. to the skin and eyes, include aqueous solutions, suspensions, ointments, creams, gels or sprayable formulations, for example, for delivery by aerosol or the like.

Said compositions for the administration of the compounds of present invention may conveniently be presented in suitable dosage unit form and may be prepared by any of the methods well known in the art depending upon various factors such as physicochemical properties, pharmacokinetic profile of drugs etc. All methods include the step of bringing the active ingredient into association with the carrier which constitutes one or more accessory ingredients. For example direct compression, granulation, spheronization etc for oral preparation..

The dosage of the active compounds can be selected based on a variety of factors, such as mode of administration, age and/or patient condition. Preferred dosages for the active ingredients of the pharmaceutical combination according to the present invention are therapeutically effective dosages, especially those which are used clinically.

The following examples illustrate the fixed dose composition according to present invention; however, it is not intended to restrict the scope of this invention in any manner.

### Examples:

**Ex.1 Tablet**

| Active Ingredient of General formula I | Therapeutically effective amount |
|---|---|
| Metformin HCL | 500mg |
| Lactose | 100mg |
| Microcrystaline Cellulose | 51mg |
| Starch | 60mg |
| Polyvinyl pyrolidone (K-30) | 2mg |
| Magnesium Stearate | 1mg |
| Purified Water | Q.S. |

| | |
|---|---|
| 1. Sift active compound of formula (I), metformin HCL, starch, lactose and microcrystalline cellulose and mix thoroughly. 2. Dissolve PVP K30 in purified water and granulate the blend of step-1 with binder solution. 3. Dry the granules and mill them through suitable screen. 4. Sift magnesium stearate and mix with dried granules. 5. Compress the lubricated granules from step-4 in to tablets. | |

**Ex. 2 Tablet**

| Active Ingredient of General formula I | Therapeutically effective amount |
|---|---|
| Amlodipine Besylate | 7mg |
| Calcium Hydrogen Phosphate | 63mg |
| Microcrystaline Cellulose | 124mg |
| Sodium Starch Glycolate | 4mg |
| Magnesium Stearate | 2mg |

| | |
|---|---|
| 1. Sift active compound of formula (I), amlodipine besylate, calcium hydrogen phosphate and microcrystalline cellulose and mix thoroughly. 2. Sift sodium starch glycolate and mix with blend of step-1. 3. Sift magnesium stearate and mix with blend of step-2. 4. Sift magnesium stearate and mix with dried granules. 5. Compress the lubricated blend from step-3 in to tablets. | |

**Ex. 3 Film coated Tablet**

| Active Ingredient of General formula I | Therapeutically effective amount |
|---|---|
| Atorvastatin | 10mg |
| Calcium Carbonate | 33mg |
| Microcrystaline Cellulose | 60mg |
| Lactose | 33mg |
| Crosscarmellose Sodium | 9.75mg |
| Polysorbate 80 | 0.5mg |
| Hydroxy Propyl Cellulose | 3mg |
| Magnesium Stearate | 0.75mg |

| Film Coating | |
|---|---|
| Opadry white | 6mg |
| Simeticone emulsion | 0.1 |
| Purified Water | Q.S. |

| | |
|---|---|
| 1. Sift active compound of formula (I), atorvastatin, calcium carbonate, microcrystalline cellulose, lactose and part quantity of crosscarmellose sodium and mix thoroughly. 2. Disoolve polysorbate 80 in purified water. Add hydroxypropyl cellulose in surfactant solution. Add this binder solution to the blend of step-1 and granulate. 3. Dry the granules and mix them through screen. 4. Sift remaining quantity of crosscarmellose sodium ad mix with dried granules. 5. Sift magnesium stearate and mix with blend of step-4. 6. Compress the lubricated blend from step-5 in to tablets. 7. Prepare coating suspension by dispersing Opadry® white in purified water and add simeticone emulsion. Coat the tablets with this coating suspension. | |

**Ex. 4 Capsule**

| Active Ingredient of General formula I | Therapeutically effective amount |
|---|---|
| Metformin HCL | 500mg |
| Indapamide | 2.5mg |
| Microcrystaline Cellulose | 51mg |
| Lactose | 100mg |
| Starch | 60mg |
| Polyvinyl pyrolidone (K-30) | 2mg |
| Magnesium Stearate | 1mg |
| Purified Water | Q.S. |

Follow the same procedure of example-1.

### Biological Examples:

### To study the effect of compound 27 on diabetic cardiomyopathy and nephropathy in animal model.

### Example-1:

**Aim:** The aim of this study was to study the effect of compound 27 on diabetic cardiomyopathy and nephropathy in obese Zucker spontaneously hypertensive fatty rats (Ob-ZSF1), an animal model of diabetes with progressive cardiac and renal dysfunction.

**Method:** Male Ob-ZSF1 rats were implanted with telemetry transmitters. At the age of 3 months, rats were divided into two treatment groups, compound 27, 9mg/kg ip bid and 27mg/kg ip, bid and a control group to which vehicle was administered until study termination at 34 weeks. 0.5% salt was added in drinking water from 29^{th} week of treatment for 6 weeks in order to accelerate the vasculopathy. At fixed predetermined time intervals, blood pressure, urine albumin and creatinine were monitored during the study. Terminal measurement of cardiac function was performed in the same animals using Millar Pressure Volume (P-V) System. PV loops were captured to establish cardiac functional parameters and differences in end systolic pressure volume relationship (ESPVR) and end diastolic pressure volume relationship (EDPVR) at different preloads. Heart and kidney were collected from same set of animals at termination for gross and microscopic histopathological examination and for gene expression studies.

**Results:** Evaluation of cardiac function revealed significant improvement in cardiac output, better systolic contractile function, more efficient diastolic relaxation and improved ventricular compliance in compound 27 treated groups. Also observed with treatment, was a reduction in afterload as indicated by a significant reduction in peripheral resistance, which could have contributed to the reduction in BP and improvement in cardiac function observed in the compound 27 treated animals. The markers of cardiac failure studied revealed that Brain natriuretic peptide (BNP) expression was reduced in the compound 27-treated group as compared to control animals. Inflammatory cytokine Interleukin-6 (IL-6) was also reduced significantly in compound 27 treated groups. Relative heart wet weight were significantly lesser in the compound 27, 27mg/kg group and microscopic examination revealed less severe damage to cardiomyocytes as compared to the control group.

Renal function also showed distinct improvement in the higher dose compound 27 treated group as compared to control. Percent increase from baseline for urinary albumin excretion, serial creatinine excretion and albumin to creatinine ratio, longitudinally over time, was significantly reduced in the compound 27 treated group as compared to control which also correlated well with less severe renal pathology observed microscopically.

**Conclusions:** In this study, compound 27, an AGE-breaker, clearly preserved cardiac function and reduced the severity of renal dysfunction in Ob-ZSF1, an animal model with persistent severe hyperglycemia leading to diabetic cardiomyopathy and nephropathy.

### Example-2:

### Effect of compound 27 on HF as an add-on therapy

Study Title: Evaluation of Safety and Efficacy of compound 27 in the treatment of stable heart failure associated with HbAlc ≥ 6.5% or type 2 Diabetes receiving oral hypoglycaemic therapy (with or without additional insulin) as an add-on therapy to conventional treatment for heart failure.

Study would be conducted in different centres in India and Europe. Sufficient number of patients would be recruited to ensure that about 300 patients will be evaluated at the completion of study. The duration of the study for the individual subject will be about 54 weeks, including 48 weeks of treatment.

### Objectives:

To evaluate the safety and efficacy of compound 27 in patients of stable Heart Failure (HF) associated with impaired blood glucose levels as an add-on therapy to existing medications, particularly one or more agent selected from the group consisting of antihypertensive, antidiabetic, hypolipidemic, antithrombotic, antiobesity, antiplatelet, an agents for treatment of diabetic vascular complications or agent for treatment for HF and to define the recommended dose level for further pivotal studies.

### Methodology:

This will be a randomized, double-blind, multinational, multi-centre, placebo-controlled, parallel-group study. Subjects will be divided into five groups of 60 subjects each, i.e. 3 groups would be receiving compound 27 at 3 different doses level and 2 groups would receive placebo for entire study duration.

### Main Criteria for Inclusion:

- Subjects of either sex, aged >45 years
- Subjects with chronic heart failure (NYHA class II - III) as established by criteria defined stable on current medications for past 3 months.
- Subjects with Type 2 Diabetes Mellitus (i.e. receiving oral therapy with or without insulin) or an impaired glucose tolerance (HbA1c should be ≥ 6.5% -10.0% at screening)
- Subjects with an NT-pro BNP ≥ 600 pg/mL (subjects with atrial fibrilation NT-pro BNP ≥ 1200 pg/mL)
- Subjects receiving atleast loop or thiazide diuretic for treating HF
- Subjects able to undergo cardiopulmonary exercise testing

### Study endpoints:

Primary efficacy endpoints
- Physical dimension of Minnesota Living with Heart Failure Questionnaire (MLWHFQ)
- Oxygen uptake efficiency slope (OUES)

Secondary efficacy endpoints
- NT-pro BNP (N-terminal pro brain natriuretic peptide) levels
- Significant & persistent change in diuretic dosage, anytime during the therapy persistence: 2 weeks
   dose changes: loop diuretics - ≥ 40 mg furosemide and ≥ 1 mg bumetanide
   torasemid ≥ 10 mg
   or change from thiazides to loop diuretics
   or addition of thiazides or addition of furosemide to loop diuretic
- Impedance cadiography (ICG) parameters
- Tissue Doppler echocardiography (at rest)
- Oxygen Cost Diagram (OCD)

Primary safety variables
- Morbidity/hospitalization for cardiac reasons
- Mortality
- Glomerular filtration rate (eGFR)

Secondary safety variables
- Daily weight
- Safety laboratory parameters
- Adverse events and concomitant medication

### Statistical Methods:

Formal statistical comparisons between treatments will be performed for the primary efficacy endpoints only for the results obtained after 26 weeks and 50 weeks of randomised treatment.

The change vs. baseline of the physical dimension of MLWHFQ and the OUES will be compared between treatments by statistical analysis and all other parameters will be evaluated descriptively.

The most preferred embodiments of the invention are :
✔ A pharmaceutical combination comprising a compound represented by formula (I) and/or (II) as defined above or its pharmaceutically acceptable salt thereof, and one or more compound selected from a) antihypertensive agent b) hypolipemic agent c) antidiabetic agent d) antiplatelet agent e) anti-thrombotic agent f) drug for diabetic vascular complications or a pharmaceutically acceptable salts thereof, optionally in presence of a pharmaceutically acceptable excipient.
✔ A kit comprising in separate containers in a single package a pharmaceutical compositions comprising in one container a pharmaceutical composition comprising a compound of formula (I) and/or (II) as defined above or its pharmaceutically acceptable salt thereof, and in a second container a pharmaceutical composition comprising one or more compound selected from a) antihypertensive agent b) hypolipemic agent c) antidiabetic agent d) antiplatelet agent e) anti-thrombotic agent f) drug for diabetic vascular complications or a pharmaceutically acceptable salts thereof.
✔ A package comprising a compound of formula (I) and/or (II) as defined above or its pharmaceutically acceptable salt thereof, together with instructions for use in combination with one or more compound selected from a) antihypertensive agent b) hypolipemic agent c) antidiabetic agent d) antiplatelet agent e) anti-thrombotic agent f) drug for diabetic vascular complications or a pharmaceutically acceptable salts thereof.
✔ A pharmaceutical combination or composition comprising: (a) compound of formula (I) and/or (II) as defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier. The composition may also be in a form of kit.

Preferred embodiments of the invention are defined in the following paragraphs:
1. A pharmaceutical combination comprising a compound represented by formula (I) or its pharmaceutically acceptable salt, 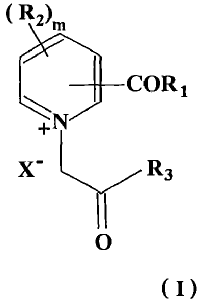 Wherein;
   R₁ is -R₄-R₅ or -N(R₇) N (R₇) R₉;
   R₄ is selected from the group consisting of -N(R₇)R₆O-, -N(R₇)R₆N(R₇)-, OR₆O,
   and -OR₆N(R₇)-, where R₆ is alkyl with C₂ to C₈ carbon atoms; R₅ is selected from the group consisting of alkyl, aryl including heteroaryl, -COR₇, SO₂R₇, -C(S) NHR₇,-C(NH)NHR₇, -COR₁₀, 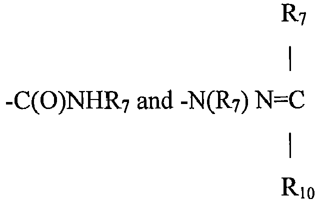 where R₇ is selected from the group consisting of H, alkyl and aryl including heteroaryl provided R₇ may be the same or different for R₁ and R₃ in the same compound;
   R₂ is selected from the group consisting of F, Cl, Br, I, OR₇, NO₂, alkyl, aryl including heteroaryl, formyl, acyl, C(O)NR₇R₁₀, C(O)OR₇, NR₇R₁₀, N=C(R₇)(R₁₀), SR₇, SO₂NH₂, SO₂ alkyl and SO₂aryl,
   and m is 0, 1 or 2;
   R₃ is selected from the group consisting of R₇, OR₇, N(R₇) (R₁₀), N=C(R₇) (R₁₀), N(R₇) N(R₇) (R₁₀), N(R₇) N=C(R₇) (R₁₀) and CH(R₇)C(O)R₈;
   where R₈ is selected from the group consisting of R₇, OR₇ and NR₇R₁₀;
   R₉ is selected from the group consisting of hydrogen, alkyl, aryl including heteroaryl, C(O)R₁₀, -SO₂R₁₀, -C(S)NHR₁₀, -C(NH) NH (R₁₀) and -C(O) NHR₁₀,
   R₁₀ is selected for the group consisting of H, alkyl or aryl including heteroaryl and in each case may be the same or different from substituent R₇, provided R₁₀ may be the same or different for R₁ and R₃ in the same compound;
   X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, BF₄⁻ and PF₆⁻ or null;
   with proviso that,
   (i) when two alkyl groups are present on the same carbon or nitrogen, they may be linked together to form a cyclic structure ;
   (ii) the nitrogen of heteroaryl ring of R₁₀, when present, may be quaternized;
   (iii) when R₃ is OR₇ and R₁ is -NHNH₂ then R₇ is not alkyl and
   (iv) when R₃ is OR₇, R₁ is N(R₇)(NR₇)R₉ and R₉ is C(O)R₁₀ where
      R₁₀ is alkyl, then R₇ is not hydrogen.
   and/or a compound represented by formula (II) or its pharmaceutically acceptable salt thereof, 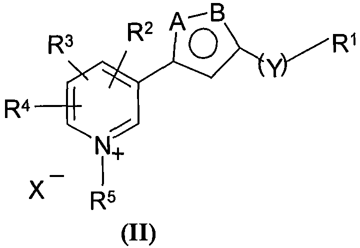 wherein,
   R¹ is alkyl or aryl group;
   Y is selected from the group comprising of sulfur, oxygen, nitrogen, or alkylene;
   A and B are independently selected from nitrogen, NH, NR6, sulfur, oxygen or carbon to form heteroaromatic ring system;
   R², R³ and R⁴ are independently selected from the group consisting of F, Cl, Br, I, OR⁷, NO₂, alkyl, aryl including heteroaryl, formyl, acyl, C(O)NR⁶R⁷, C(O)OR⁶, NR⁶R⁷, N=C(R⁶)(R⁷), SR⁶, SO₂NH₂, SO₂alkyl, SO₂aryl; R², R³ and R⁴ might be optionally joined together to form a ring system;
   R⁵ is independently selected for the group consisting of alkyl, aryl and null;
   R⁶ is independently selected from the group consisting of H, alkyl and aryl including heteroaryl provided R⁶ might be different for R², R³ and R⁴ in the same compound;
   R⁷ is independently selected from the group consisting of H, alkyl and aryl including heteroaryl and in each case optionally different from substituent R⁶, provided R⁷ might be different for R², R³ and R⁴ in the same compound;
   X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogenion; phosphonate ion, phosphate ion, BF4, PF6 and null;
   with proviso that when two alkyl groups are present on the same carbon or nitrogen, they are optionally linked together to form a cyclic structure;
   and atleast one therapeutic agent selected from the group consisting of: a) antihypertensive agent; b) hypolipemic agent; c) antidiabetic agent; d) antiplatelet agent; e) anti-thrombotic agent; f) antiobesity agent; g) agent for treatment of heart failure; and h) drug for diabetic vascular complications; or a pharmaceutically acceptable salts thereof.
2. A pharmaceutical composition comprising a compound represented by formula (I) and/or (II) or its pharmaceutically acceptable salt thereof, and atleast one therapeutic agent selected from the group consisting of: a) antihypertensive agent; b) hypolipemic agent; c) antidiabetic agent; d) antiplatelet agent; e) anti-thrombotic agent; f) antiobesity agent; g) agent for treatment of heart failure; and h) drug for diabetic vascular complications; or a pharmaceutically acceptable salts thereof, optionally in presence of a pharmaceutically acceptable excipient.
3. The pharmaceutical combination as defined in para 1, wherein the compound of formula (I) or (II) is selected from:
   1-(2-ethoxy -2- oxoethyl) -4-(phenylsulfonyl hydrazino carbonyl) pyridinium bromide (compound 1);
   1-(2-phenylamino-2-oxoethyl)-4- (phenylsulfonyl hydrazino carbonyl) pyridinium chloride (compound 2);
   1-(2-ethoxy -2- oxoethyl) -3-(phenylsulfonyl hydrazino carbonyl) pyridinium bromide (compound 3);
   1-(2-(2',4'-dichlorophenyl)-2-oxoethyl)-3-(2(methoxy)ethyloxycarbonyl) pyridinium bromide (Compound 4);
   1-(2-phenylamino-2-oxoethyl)-3-((benzoyloxy)ethylaminocarbonyl) pyridinium chloride (compound 5);
   1-(2-thien-2'-yl- 2-oxoethyl)-3-(phenylaminocarbonyl hydrazinocarbonyl)pyridinium bromide (compound 6);
   1-(2-phenyl-2-oxoethyl)-3-(2-(acetoxy)ethylaminocarbonyl) pyridinium bromide (compound 7);
   1-(2-phenylamino-2-oxoethyl)-3-(phenyl sulfonyl hydrazino carbonyl) pyridinium chloride (compound 8);
   1-(2-phenylamino-2-oxoethyl)-3-((4-methylphenyl) sulfonyl hydrazino carbonyl)pyridinium chloride (compound 9);
   1-(2-phenyl-2-oxoethyl)-3-(2-(benzoyloxy)ethyloxy carbonyl) pyridinium bromide (compound 10);
   1-(2-thien-2'-yl-2-oxoethyl)-3-(phenylcarbonyl hydrazino carbonyl) pyridinium bromide (compound 11);
   1-(2-ethoxy-2-oxoethyl)-3-((phenylmethyl)sulfonyl hydrazino carbonyl)pyridinium bromide (compound 12);
   1-(2-phenyl-2-oxoethyl)-3-((phenylmethyl)sulfonyl hydrazino carbonyl) pyridinium bromide (compound 13);
   N, N' - bis [3-carbonyl-1-(2-furan-2'-yl-2-oxoethyl) pyridinium] hydrazine dibromide. (Compound No: 14);
   N, N'-bis [3-carbonyl-1- (2-thien-2'-yl-2-oxoethyl) pyridinium] hydrazine dichloride.(Compound No: 15);
   1-(2-thien-2'-yl-2-oxoethyl)-3-((2-(1-oxo-3-cyclohexyl)-propyl)-hydrazino carbonyl)-pyridinium bromide.(Compound No: 16);
   1-(2-phenylamino-2-oxo ethyl)-3-({2-(1-oxo-3-cyclohexyl)-propyl}-hydrazino-carbonyl}-pyridinium bromide.(Compound No: 17);
   1-(2-thien-2'-yl-2-oxoethyl)-3-[2-(benzoyloxy)ethylamino carbonyl]-pyridinium bromide (Compound No: 18);
   1-(4-ethoxy-2, 4-dioxobutyl)-3-(2-(benzoyloxy)ethylamino carbonyl)-pyridinium chloride. (Compound No: 19);
   1-(2', 4'-dichloro-phenyl-2-oxoethyl)-3-(2-methoxyethyl aminocarbonyl)-pyridinium bromide. (Compound No: 20);
   N,N'-bis-[3-carbonyl-1-(2-cyclopropylamino-2-oxoethyl) pyridinium] hydrazine dichloride. (Compound No: 21);
   1-(2-cyclopropylamino-2-oxoethyl)-3-(2-methoxyethylaminocarbonyl)-pyridinium chloride. (Compound No: 22);
   N-N'-bis [3-carbonyl-1-(2-isopropylamino-2-oxoethyl) pyridinium] hydrazine dichloride. (Compound No: 23);
   1-(2-thien-2'yl-2-oxoethyl)-3-(2-(2-chloro-3-pyridoylhydrazinocarbonyl) -pyridinium chloride. (Compound No: 24);
   1-(2-isopropylamino-2-oxoethyl)-3-(2-methylsulfonylhydrazinocarbonyl) -pyridinium chloride. (Compound No: 25);
   1-(2-(1-pyrrolidinyl)-2-oxoethyl)-3-(methanesulfonyl hydrazino carbonyl) pyridinium chloride. (Compound No: 26);
   1-(2-thien-2'-yl-2-oxoethyl)-3-(methanesulfonyl hydrazino carbonyl) pyridinium chloride. (Compound No: 27);
   N,N'-bis[3-carbonyl-1-(2-hydroxy-2-oxoethyl)pyridinium]hydrazine dichloride (Compound No: 28);
   1-(2-thien-2'-yl-2-oxoethyl)-3-((2-methoxy ethyl) amino carbonyl)-5-bromo pyridinium chloride (Compound No: 29);
   1-(2'-thien-2'-yl-2-oxoethyl)-3-[1-oxo-1-(2-methoxy carbonyl)pyridyl] hydrazino pyridinium chloride. (Compound No: 30);
   1-[1-(2-thien-2'-yl-2-oxoethyl)-6-methyl-3-carbonyl pyridinium]-2-[1-(2-Thien-2'-yl-2-oxoethyl)-3-carbonyl pyridinium] hydrazine dichloride(compound no: 31);
   1-(2-thien-2'-yl-2-oxoethyl)-3-(isopropylsulfonyl hydrazino carbonyl) pyridinium bromide(compound no: 32);
   1-(2-(4-benzyl piperidin-1-yl)-2-oxoethyl)-3-(methanesulfonyl hydrazino carbonyl) pyridinium chloride(compound no: 33);
   1-(2-(2-ethoxy carbonyl pyrrolidin-1-yl)-2-oxoethyl)-3-(methanesulfonyl hydrazino carbonyl) pyridinium chloride. (compound no: 34);
   1-(2-thien-2'-yl-2-oxoethyl)-3-(methanesulfonyl hydrazino carbonyl)-5-bromo pyridinium bromide. (compound no: 35);
   1-(2-thien-2'-yl-2-oxoethyl)-3-(ethoxycarbonyl hydrazino carbonyl) pyridinium bromide. (compound no: 36);
   1-(2-(5-chloro-thien-2-yl)-2-oxoethyl)-3-(methanesulfonyl hydrazino carbonyl ) pyridinium bromide (compound no: 37);
   N,N'-bis[3-carbonyl-1-(2-(4-nitro-thien-2-yl)-2-oxoethyl)pyridinium] hydrazine dichloride. (compound no: 38);
   1-(2-thien-2'-yl-2-oxoethyl)-3-(methanesulfonyl hydrazino carbonyl ) -6-methyl pyridinium bromide. (compound no: 39);
   N,N'-bis[3-carbonyl-1-(2-(5-methyl-thien-2-yl)-2-oxoethyl) pyridinium] hydrazine dichloride. (compound no: 40);
   N,N'-bis[3-carbonyl-1-(2-(2-ethoxycarbonyl pyrrolidin-1-yl)-2-oxoethyl) pyridinium] hydrazine dichloride. (compound no: 41);
   1-[1-(2-thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 42);
   1-(2-(4-carbethoxy-thiazolidin-3-yl)-2oxoethyl)-3-(methanesulfonyl hydrazino carbonyl) pyridinium chloride (compound no: 43);
   N,N'-bis[3-carbonyl-1-(2-(5-chloro-thien-2-yl)-2-oxoethyl) pyridinium] hydrazine dichloride. (compound no: 44);
   1-(2-(5-methyl-thien-2-yl)-2-oxoethyl)-3-(methanesulfonyl hydrazino carbonyl) pyridinium chloride (compound no: 45);
   1-(2-(4-nitro-thien-2-yl)-2-oxoethyl)-3-(methanesulfonyl hydrazino carbonyl) pyridinium bromide. (compound no: 46);
   1-(2-phenylamino-2-oxoethyl)-3-(phenyl hydrazino carbonyl) pyridinium chloride (compound no: 47);
   1-(2-phenylamino-2-oxoethyl)-4 -[2-(benzoyloxy) ethylamino carbonyl] pyridinium chloride (compound no: 48);
   1-2-(5-nitro-thien-2-yl)-2-oxoethyl)-3-(methanesulfonyl hydrazino carbonyl) pyridinium chloride (compound no: 49);
   1-(2-thien-2'-yl-2-oxoethyl)-3-(trifluoromethanesulfonyl hydrazino carbonyl) - pyridinium bromide. (compound no. 50);
   1-(2-thien-2'-yl-2-oxoethyl)-3-(phenyl hydrazino carbonyl) pyridinium bromide (compound no.51);
   1-(2-thien-2'-yl-2-oxoethyl)-3-(p-methoxy phenyl sulfonyl hydrazino carbonyl) pyridinium bromide (compound no. 52);
   1-(2-ethoxy-2-oxoethyl)-3-(phenyl aminocarbonyl hydrazino carbonyl) pyridinium bromide (compound no. 53);
   1-(2-ethoxy-2-oxoethyl)-3-(p-toluene sulfonyl hydrazino carbonyl) pyridinium bromide (compound no. 54);
   1-(2-phenyl-2-oxoethyl)-3-(phenylamino carbonyl hydrazino carbonyl) pyridinium bromide (compound no. 55);
   1-(2-phenylamino-2-oxoethyl)-3-(benzyl sulfonyl hydrazino carbonyl) pyridiniumchloride. (compound no. 56);
   1-(2-phenyl-2-oxoethyl)-4-(methanesulfonyl hydrazino carbonyl) pyridinium bromide (compound no. 57);
   1-(2-phenyl-2-oxoethyl)-3-(phenyl hydrazino carbonyl) pyridinium bromide. (compound no. 58);
   1-(2-ethoxy-2-oxoethyl)-4-[2-(benzoyloxy) ethyl amino carbonyl] pyridinium bromide (compound no. 59);
   1-(2-ethoxy-2-oxoethyl)-3-(phenyl hydrazino carbonyl) pyridinium bromide.(compound no. 60);
   1-(2-phenyl-2-oxoethyl)-3-(p-methoxyphenyl sulfonyl hydrazino carbonyl) pyridinium bromide. (compound no. 61);
   1-(2-phenyl-2-oxoethyl)- 4-[2-(benzoyloxy) ethyl amino carbonyl] pyridinium bromide. (compound no. 62);
   1-(2-ethoxy-2-oxoethyl)- 4-(p-methanesulfonyl hydrazino carbonyl) pyridinium bromide. (compound no. 63);
   N, N'-Bis [3-carbonyl-1- (2-phenyl-2-oxoethyl)-pyridinium] hydrazine dibromide (compound 64);
   N, N'-Bis [3-carbonyl-1-(2-ethoxy-2-oxoethyl) pyridinium] hydrazine dibromide (compound 65);
   N, N'-Bis [3-carbonyl-1- (2- (2, 4-dichlorophenyl)-2-oxoethyl) pyridinium] hydrazine dibromide (compound 66);
   1- (2-Ethoxy-2-oxoethyl)-3- (2- (2-pyridyl) hydrazinocarbonyl) pyridinium bromide (compound 67);
   1- (2-Thien-2'-yl-2-oxoethyl)-3- (methanesulfonyl hydrazinocarbonyl) pyridinium bromide (compound 68);
   N, N'-Bis [3-carbonyl-1- (2-thien-2'-yl-2-oxoethyl) pyridinium] hydrazine dibromide (compound 69);
   1- (2-Ethoxy-2-oxoethyl)-3- (2- (benzoyloxy) ethylaminocarbonyl) pyridinium bromide (compound 70);
   1- (2- (2,4-Dichlorophenyl)-2-oxoethyl)-3- (2-(benzoyloxy) ethylamino- carbonyl) pyridinium bromide (compound 71);
   1- (2-Thien-2'-yl-2-oxoethyl)-3- (2- (2-pyridyl) hydrazinocarbonyl) pyridinium bromide (compound 72);
   1- (2-Phenyl-2-oxoethyl)-3- (2- (2-pyridyl) hydrazinocarbonyl) pyridinium bromide (compound 73);
   1- (2-Phenyl-2-oxoethyl)-3- (hydrazinocarbonyl) pyridinium bromide (compound 74);
   1- (2- Phenyl-2-oxoethyl)-3- (methanesulfonyl hydrazinocarbonyl) pyridinium bromide (compound 75);
   1- (2-Ethoxy-2-oxoethyl)-3- (methanesulfonyl hydrazinocarbonyl) pyridinium bromide (compound 76);
   1- (2-Phenyl-2-oxoethyl)-3- (phenylsulfonylhydrazino carbonyl) pyridinium bromide (compound 77);
   1- (2-Phenyl-2-oxoethyl)-2-chloro-3- (phenylsulfonylhydrazino carbonyl) pyridinium bromide (compound 78);
   1- (2- Phenyl-2-oxoethyl)-3- (2- (methoxy) carbonyl) ethyloxy carbonyl pyridinium bromide (compound 79);
   1- (2-Ethoxy-2-oxoethyl)-3- (2- (benzoyloxy) ethyloxy carbonyl) pyridinium bromide (compound 80);
   1-(2-Thien-2'-yl-2-oxoethyl)-4-(2-(benzoyloxy) ethylaminocarbonyl) pyridinium bromide (compound 81);
   1- (2-thien-2'-yl-2-oxoethyl)-3- [(3-phenyl methyl)pyrazol-5-yl] pyridinium bromide (compound 82);
   1-(2-thien-2'-yl-2-oxoethyl)-3-[(3-phenyl methyl) oxazol-5-yl pyridinium bromide (compound 83);
   1-(2-thien-2'-yl-2-oxoethyl)-3-[3-{1-(2-thien-2'-yl)-2-oxoethyl pyridinium-4 thio} methyl-pyrazol-5-yl] pyridinium dibromide. (compound 84);
   1- (2-thien-2'-yl-2-oxoethyl)-3- [3- f 1- (3, 5-dimethylpyrazol-1-yl) methyl} pyrazol-5-yl] pyridinium bromide. (compound 85);
   1- (2-thien-2'-yl-2-oxoethyl)-3- [3-phenylmethyl}-1-{2-pyridyl}-pyrazol-5-yl]-pyridinium bromide. (compound 86);
   1- (2-thien-2'-yl-2-oxoethyl)-3- [3 (3, 5-dimethylpyrazol-1-yl) methyl-1-pyridyl} pyrazol-5-yl] pyridinium bromide. (compound 87);
   1- [2- (cyclopropylamino)- 2-oxoethyl] 3- [3- (3, 5-dimethylpyrazol-1-yl) methyl}-pyrazol-5-yl]-pyridinium bromide. (compound 88);
   1- {2-(4-nitro-2-thienyl)-2-oxoethyl}-3-[3 {(3,5-dimethylpyrazol-1-yl) methyl} pyrazol-5-yl]-pyridinium bromide. (compound 89);
   1- (2-cyclopropylamino-2-oxoethyl)-3 [(3-phenylmethyl) pyrazol-5-yl] pyridinium chloride. (compound 90);
   3,5-bis-[1-(2-thien-2'-yl-2-oxoethyl)-pyridinium-3-yl]-pyrazole dibromide. (compound 91);
   1-(2-thien-2'-yl-2-oxoethyl)-3-1 (1-phenyl-3-phenylmethyl) pyrazol-5-yl] pyridinium chloride. (compound 92);
   1-(2-(5'-methyl-2-thienyl)-2-oxoethyl)-3-[(3-phenylmethyl) pyrazol-5-yl] pyridinium chloride. (compound 93);
   1- (2-thien-2'-yl-2-oxoethyl) 3- [1-phenyl, 3- { (3, 5-dimethyl pyrazol-1-yl) methyl)} pyrazol-5-yl]-pyridinium chloride. (compound 94);
   1- (2-phenyl-2-oxoethyl)-3- [(3-phenylmethyl) pyrazol-5-yl]-pyridinium bromide. (compound 95);
   1- (2-cyclopropylamino- 2-oxoethyl) 3- [(1-phenyl-3-phenylmethyl) pyrazol-5 yl]-pyridinium chloride (compound 96);
   1- (2- (4-benzyl-l-piperidinyl)-2-oxoethyl) 3- [(3-phenoxymethyl) pyrazol-5-yl] pyridinium bromide (compound 97);
   1 (2-phenyl-2-oxoethyl)-3-[(3-(3, 5-dimethylpyrazol-1-yl) methyl) pyrazol-5 yl]-pyridinium chloride (compound 98);
   1-(2-(5-methyl-2-thienyl)-2-oxoethyl)-3-[(3,-(3, 5-dimethylpyrazol-1-yl) methyl). pyrazol-5-yl] pyridinium chloride (compound 99);
   1- (2-phenyl-2-oxoethyl)-3- [(1-phenyl-3-phenylmethyl) pyrazol-5-yl] pyridinium chloride (compound 100);
   1- (2- (5-methyl-2-thienyl)-2-oxoethyl)-3- [(3 (2-cyclohexyl ethyl) pyrazol-5-yl] pyridinium chloride (compound 101);
   1- (2-cyclopropylamino-2-oxoethyl)-3- [(3- (2-cyclohexylethyl) pyrazol-5-yl] pyridinium chloride (compound 102);
   1- (2-phenyl-2-oxoethyl)-3- [(3-(2-cyclohexylethyl) pyrazol-5-yl] pyridinium chloride (compound 103);
   1- (2-cyclopropylamino-2-oxoethyl)-3- [(1-cyclohexyl-3-phenylmethyl) pyrazol 5-yl] pyridinium chloride (compound 104);
   1- (2-thien-2'-yl-2-oxoethyl)-3- [(3-phenoxymethyl) pyrazol-5-yl] pyridinium chloride (compound 105);
   1- [2- (l-adamantylamino)-2-oxoethyl]-3- [(3-phenylmethyl) pyrazol-5-yl] pyridinium chloride (compound 106);
   1- (2-phenyl-2-oxoethyl)-3- [{3- (3, 5-dimethylpyrazol-1-yl) methyl)} 1-phenyl-pyrazol-5-yl] pyridinium bromide (compound 107);
   1-(2-(4-nitor-2-thienyl)-2-oxoethyl)-3-[(1-cyclohexyl-3-(3,5-dimethylpyrazol-1yl)-methyl) pyrazol-5-yl] pyridinium bromide (compound 108);
   1- (2- (4-nitro-2-thienyl)-2-oxoethyl)-3 [(3- (2-cyclohexylethyl) pyrazol-5-yl] pyridinium bromide (compound 109);
   1-(2-thien-2'-yl-2-oxoethyl)-3-[(1-phenyl-3-phenoxymethyl) pyrazol-5-yl] pyridinium chloride (compound 110);
   1- (2- (4-nitro-2-thienyl)-2-oxoethyl)-3- [(1-phenyl-3-phenylmethyl) pyrazol-5 yl] pyridinium bromide (compound 111);
   pyrazolel- (2-cyclopropylamino-2-oxoethyl)-3- [(3-phenoxymethyl) pyrazol-5-yl] pyridinium chloride (compound 112);
   1- (2-cyclopropylamino-2-oxoethyl)-3- [(1-cyclohexyl-3- (3, 5-dimethyl pyrazole)-1-yl)-5-yl] pyridinium chloride (compound 113);
   1- (2- (5-chloro-2-thienyl)-2-oxoethyl)-3- [(3-phenoxymethyl) pyrazol-5-yl] pyridinium bromide (compound 114);
   1-(2-phenyl-2-oxoethyl)-3-[(1-phenyl-3-phenoxyrnethyl) pyrazol-5-yl] pyridinium chloride (compound 115);
   1- (2-thien-2'-yl-2-oxoethyl)-3- [(1-cyclohexyl-3- (3, 5-dimethylpyrazol-1-yl-methyl) pyrazol-5-yl] pyridinium chloride (compound 116);
   1- (2-cyclopropylamino-2-oxoethyl)-3- [(1-phenyl-3-phenoxymethyl) pyrazol-5 yl] pyridinium bromide (compound 117);
   1- (2-thien-2'-yl-2-oxoethyl)-3- [(1-phenyl-3-(2-cyclohexylethyl)pyrazol-5-yl] pyridinium bromide (compound 118);
   1-(2-thien-2'-yl-2-oxoethyl)-3-[(1-cyclohexyl-3-phenoxymethyl)pyrazol-5-yl] pyridinium chloride (compound 119);
   3- [(3-phenylmethyl) pyrazol-5-yl] pyridine hydrochloride (compound 120);
   3- [(3-phenoxymethyl) pyrazol-5-yl] pyridine hydrochloride (compound 121);
   3- [(3, 5-dimethylpyrazol-1-yl-methyl) pyrazol-5-yl] pyridine (compound 122);
   3- [3- (2-cyclohexyl-ethyl)-pyrazol-5-yl] pyridine (compound 123);
   1- (2-napthyl-2-oxo ethyl)-3 [(3-phenoxymethyl) pyrazol-5-yl] pyridinium bromide (compound 124);
   1- (phenylmethyl)-3 [(3-phenyl methyl)pyrazol-5-yl] pyridinium chloride (compound 125);
   1-(2-thien-2'-yl-2-oxo ethyl)-3[(3 (-1-naphthyl) pyrazol-5-yl] pyridinium chloride (compound 126);
   1- (2-phenyl-2oxoethyl)-3 [3 (thienyl-2-yl-methyl)pyrazol-5-yl] pyridinium chloride (compound 127);
   1- (2- (5-methyl-2-thienyl)-2-oxoethyl)-3- [3 (2-phenyl ethyl) pyrazol-5 yl] pyridinium chloride (compound 128);
   1- (2- (5-methyl 2-thienyl)-2-oxo ethyl)-3- [3- (3-phenoxy propyl) pyrazol-5 yl] pyridinium chloride (compound 129);
   1- (isopropyl)-3 [(3-phenylmethyl) pyrazol-5-yl] pyridinium bromide (compound 130);
   1- (2- (5-methyl-2-thienyl)-2-oxoethyl)-3- [(3-thiophenylmethyl) pyrazol-5 yl] pyridinium chloride (compound 131);
   1- (2-thien-2'-yl-2-oxoethyl)-3 [(3- (N-methyl-indole-3-yl methyl) pyrazol-5 yl] pyridinium chloride (compound 132);
   1- (2-napthyl-2-oxo-ethyl)-3 [(3-methyl) pyrazol-5-yl] pyridinium bromide (compound 133);
   1- (2- (1, 4 benzodioxane-6-yl-amino-2-oxoethyl)-3 [(3-phenylmethyl) pyrazol-5-yl] pyridinium chloride (compound 134);
   1- (2-thien-2'-yl-2-oxo. ethyl)-3[(3-phenyl) pyrazol-5-yl]-5 bromo pyridinium chloride (compound 135);
   1- (2-thien-2'-yl)-2-oxoethyl)-3 [(3-phenyl) pyrazol-5-yl] quinolinium chloride (compound 136) and

      3- [(3-phenyl) pyrazol-5-yl)] quinoline (compound 137).
4. The pharmaceutical combination comprising:
   1-(2-thien-2'-yl-2-oxoethyl)-3-(methanesulfonyl hydrazino carbonyl) pyridinium chloride or its pharmaceutically acceptable salts and atleast one therapeutic agent selected from the group consisting of: a) antihypertensive agent; b) hypolipemic agent; c) antidiabetic agent; d) antiplatelet agent; e) anti-thrombotic agent; f) antiobesity agent; g) agent for treatment of heart failure; and h) drug for diabetic vascular complications; or a pharmaceutically acceptable salts thereof, optionally in presence of a pharmaceutically acceptable excipient.
5. The pharmaceutical combination comprising:
   1-(2-thien-2'-yl-2-oxoethyl)-3-(methanesulfonyl hydrazino carbonyl) pyridinium chloride or its pharmaceutically acceptable salts and atleast one therapeutic agent selected from metformin, glimepiride, pioglitazone, sitagliptin, vildagliptin, amlodipine, felodipine, diltiazem, lercanidipine, ramipril, enalapril, lisinopril, perindopril, atorvastatin, simvastatin, rosuvastatin, pravastatin, fluvastatin, aliskiren, nicorandil, indapamide, clopidogrel, prasugrel, aspirin, hydrochlorothiazide, rivaroxaban, indapamide and furosemide, or its pharmaceutically acceptable salts thereof.
6. The pharmaceutical combination of para 1, wherein antihypertensive agent is selected from the group consisting of: an angiotensin converting enzyme (ACE) inhibitor; a renin inhibitor; a beta adrenergic receptor blocker; an alpha adrenergic receptor blocker; a calcium channel blocker; a potassium channel activator; an aldosterone synthase inhibitor; a neutral endopeptidase (NEP) inhibitor; a dual angiotensin converting enzyme/neutral endopeptidase (ACE/NEP) inhibitor; an endothelin receptor antagonist; a dual angiotensin and endothelin receptor antagonist (DARA); and a diuretic; or a pharmaceutically acceptable salts thereof.
7. The pharmaceutical combination of para 6, wherein angiotensin converting enzyme inhibitor is captopril, enalapril, benazepril, lisinopril, quinapril, ramipril, fosinopril, parindopril or a pharmaceutically acceptable salts thereof.
8. The pharmaceutical combination of para 6, wherein rennin inhibitor is aliskiren or its pharmaceutically acceptable salts thereof.
9. The pharmaceutical combination of para 6, wherein potassium channel activator is nicorandil or its pharmaceutically acceptable salts thereof.
10. The pharmaceutical combination of para 6, wherein calcium channel blocker is amlodipine, nicardipine, nifedipine, lercanidipine, feldipine or its pharmaceutically acceptable salts thereof.
11. The pharmaceutical combination of para 6, wherein diuretic agent is thiazide diuretic such as hydrochlorothiazide, indapamide, trichlormethazide, furosemide, chlorthalidone, altizide or a pharmaceutically acceptable salts thereof.
12. The pharmaceutical combination of para 1, wherein hypolipemic agent is selected from the group consisting of: a HMG CoA reductase inhibitor; a fibric acid derivative; a cholesterol absorption inhibitor; an ileal Na +/bile acid cotransporter inhibitor; upregulators of LDL receptor activity; a cholesteryl ester transfer protein(CETP) inhibitor; a bile acid sequestrants; and a nicotinic acid derivative; or a pharmaceutically acceptable salts thereof.
13. The pharmaceutical combination of para 12, wherein HMG CoA reductase inhibitor is simvastatin, atorvastatin, rosuvastatin, pravastatin, cerivastatin, fluvastatin, or a pharmaceutically acceptable salts thereof.
14. The pharmaceutical combination of para 12, wherein fibric acid derivative is fenofibrate, gemfibrozil, clofibrate, bezafibrate, ciprofibrate, clinofibrate, probucol, or a pharmaceutically acceptable salts thereof.
15. The pharmaceutical combination of para 12, wherein cholesterol absorption inhibitor is ezetimibe or a pharmaceutically acceptable salts thereof.
16. The pharmaceutical combination of para 1, wherein antidiabetic agent is selected from the group consisting of: a biguanide; a sulfonylurea; an alpha glucosidase inhibitor; a PPARγ agonist; a PPARα agonist; an alpha-amylase inhibitor; a fatty acid oxidation inhibitor; a PPARδ agonist or antagonist; a PPARα/γ dual agonist; a dipeptidyl peptidase IV (DP4) inhibitor; a SGLT2 inhibitor; a glycogen phosphorylase inhibitor; a glucagon-like peptide-1 (GLP-1); and meglitinide; or a pharmaceutically acceptable salts thereof, as well as insulin.
17. The pharmaceutical combination of para 16, wherein biguanide is metformin or phenformin or a pharmaceutically acceptable salts thereof.
18. The pharmaceutical combination of para 16, wherein sulfonylurea is glyburide, glimepiride, glipizide, gliclazide, chlorpropamide or a pharmaceutically acceptable salts thereof.
19. The pharmaceutical combination of para 16, wherein alpha glucoside hydrolase inhibitor is acarbose, adiposine, camiglibose, emiglitate, miglitol, voglibose, or a pharmaceutically acceptable salts thereof.
20. The pharmaceutical combination of para 16, wherein PPARγ agonist is rosiglitazone, pioglitazone, MCC-555, GL-262570, englitazone, darglitazone, isaglitazone, or a pharmaceutically acceptable salts thereof.
21. The pharmaceutical combination of para 16, wherein SGLT2 inhibitor is Sergliflozin, Dapagliflozin or a pharmaceutically acceptable salts thereof.
22. The pharmaceutical combination of para 16, wherein DPP-IV inhibitor is vildagliptin, sitagliptin, saxagliptin, alogliptin or a pharmaceutically acceptable salts thereof.
23. The pharmaceutical combination of para 16, wherein meglitinide is repaglinide, nateglinide or KAD1229 or a pharmaceutically acceptable salts thereof.
24. The pharmaceutical combination of para 1, wherein antiplatelet agent is aspirin, ticlopidine, clopidogrel, prasugrel or a pharmaceutically acceptable salts thereof.
25. The pharmaceutical combination of para 1, wherein antithrombotic agent is rivaroxban or a pharmaceutically acceptable salts thereof.
26. The pharmaceutical combination of para 1, wherein antiobesity agent is selected from the group consisting of: a 5HT (serotonin) transporter inhibitor; a NE (norepinephrine) transporter inhibitor; a CB-1 (cannabinoind-1 receptor) antagonist/inverse agonist; a H3 (histamine H3) antagonist/inverse agonist; a NPY1 (neuropeptide Y Y1) antagonist; a NPY2 (neuropeptide Y Y2) agonist; a NPY5 (neuropeptide Y Y5) antagonist; a leptin or its derivative; an opioid antagonist; 5HT2c (serotonin receptor 2c) agonist; a Mc3r (melanocortin 3 receptor) agonist; a Mc4r (melanocortin 4 receptor) agonist; a monoamine reuptake inhibitor; a β3 (beta adrenergic receptor 3) agonist; a thyroid hormone β agonist; a lipase inhibitor; a fatty acid transporter inhibitor; and a dicarboxylate transporter inhibitor; or a pharmaceutically acceptable salts thereof.
27. The pharmaceutical combination of para 1, wherein an agent for treatment of heart failure is selected from digitalis glycoside and phosphodiesterase inhibitor including pharmaceutically acceptable salts thereof.
28. The pharmaceutical combination of para 27, wherein digitalis glycoside is digitoxin, gitoxin, gitalin, digoxin, F-gitonin, digitonin, or pharmaceutically acceptable salts thereof.
29. The pharmaceutical combination of para 27, wherein phosphodiesterase inhibitor is amrinone, milrinone, enoximone, bucladesine or pharmaceutically acceptable salts thereof.
30. The pharmaceutical combination of para 1, wherein a drug for diabetic vascular complications is selected from an aldose reductase inhibitor, an AGE inhibitor and an AGE breaker or a pharmaceutically acceptable salt thereof.
31. The pharmaceutical combination of para 30, wherein AGE breaker is alagabrium or its pharmaceutically acceptable salts thereof.
32. A method of treating heart failure to a mammal in need thereof comprising administering a therapeutically effective amount of compound of formula (I) and/or (II) or a pharmaceutically acceptable salts thereof in association with pharmaceutically acceptable carrier.
33. A method of treating heart failure associated with diabetes to a mammal in need thereof comprising administering a therapeutically effective amount of compound of formula (I) and/or (II) or a pharmaceutically acceptable salt thereof in association with pharmaceutically acceptable carrier.
34. Use of compound of formula (I) and/or (II) or a pharmaceutically acceptable salts thereof in association with pharmaceutically acceptable carrier for treatment of heart failure to a mammal in need thereof.
35. Use of compound of formula (I) and/or (II) or a pharmaceutically acceptable salts thereof in association with pharmaceutically acceptable carrier for treatment of heart failure associated with diabetes to a mammal in need thereof.
36. A method of treating or preventing the disease condition related to diabetes or aging-related vascular complications by administering a pharmaceutical combination of para 1 to a mammal in need thereof, wherein the said disease condition is selected from. neuropathy (both diabetic and non-diabetic), nephropathy (both diabetic and non-diabetic), diabetic retinopathy, diabetic cardiomyopathy, hypertension, hypertensive cardiomyopathy, and dilated cardiomyopathy.
37. A method of treating or preventing the disease condition related to diabetes or aging-related vascular complications by administering a pharmaceutical combination of para 1 to a mammal in need thereof, wherein the said disease condition is selected from the group consisting of hypertension, congestive heart failure of diverse etiology like left ventricular dysfunction (systolic and diastolic), ischemic cardiomyopathy, hypertrophic cardiomyopathy, diabetic cardiomyopathy, hypertensive cardiomyopathy, dilated cardiomyopathy and metabolic cardiomyopathy (thyroid, carcinoid, pheochromocytoma or acromegaly associated), myocardial infarction and its sequelae, atherosclerosis (and complications thereof), angina (whether unstable or stable), renal insufficiency (diabetic and non- diabetic), renal failure conditions, such as diabetic nephropathy, hypertensive nephropathy, and neuropathy (both diabetic and non-diabetic).
38. Use of a pharmaceutical combination according to para 1 for treatment or prevention of diabetes and aging-related vascular complications, selected from neuropathy (both diabetic and non-diabetic), nephropathy (both diabetic and non-diabetic), diabetic retinopathy, diabetic cardiomyopathy, hypertension, hypertensive cardiomyopathy, and dilated cardiomyopathy to a mammal in need thereof.
39. Use of a pharmaceutical combination according to para 1 for treatment or prevention of the disease condition is selected from the group consisting of hypertension, congestive heart failure of diverse etiology like left ventricular dysfunction (systolic and diastolic), ischemic cardiomyopathy, hypertrophic cardiomyopathy, diabetic cardiomyopathy, hypertensive cardiomyopathy, dilated cardiomyopathy and metabolic cardiomyopathy (thyroid, carcinoid, pheochromocytoma or acromegaly associated), myocardial infarction and its sequelae, atherosclerosis (and complications thereof), angina (whether unstable or stable), renal insufficiency (diabetic and non- diabetic), renal failure conditions, such as diabetic nephropathy, hypertensive nephropathy, and neuropathy (both diabetic and non-diabetic)to a mammal in need thereof.
40. A method of an administration of pharmaceutical combination or composition comprising: (a) compound of formula (I) and/or (II) as 41. defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier, for the treatment or prevention of the disease condition related to diabetes or aging-related vascular complications, preferably congestive heart failure and more preferably diabetes associated congestive heart failure.
41. Use of pharmaceutical combination or composition comprising: (a) compound of formula (I) and/or (II) as defined above or a pharmaceutically acceptable salt thereof; (b) an antidiabetic agent; (c) a diuretic; (d) one more drugs selected from the group consisting of: an antihypertensive; a hypolipidemic; an antiplatelet; an antiobesity agent; and an antithrombotic agent; or a pharmaceutically acceptable salts thereof optionally in presence of a pharmaceutically acceptable carrier, for the treatment or prevention of the disease condition related to diabetes or aging-related vascular complications, preferably congestive heart failure and more preferably diabetes associated congestive heart failure.
42. Use of pharmaceutical combination or composition for the treatment or prevention of the disease condition related to diabetes or aging-related vascular complications, preferably congestive heart failure and more preferably diabetes associated congestive heart failure as defined in para 41, wherein the compound of formula (I) is 1-(2-thien-2'-yl-2-oxoethyl)-3-(methanesulfonyl hydrazino carbonyl) pyridinium chloride.

## Claims

1. A pharmaceutical combination comprising a compound represented by formula (I) or its pharmaceutically acceptable salt, 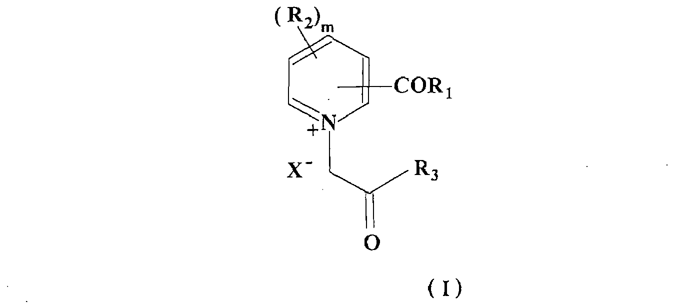 Wherein;
R₁ is -R₄-R₅ or -N(R₇) N (R₇) R₉;
R₄ is selected from the group consisting of -N(R₇)R₆O-, -N(R₇)R₆N(R₇)-, OR₆O,
and -OR₆N(R₇)-, where R₆ is alkyl with C₂ to C₈ carbon atoms; R₅ is selected from the group consisting of alkyl, aryl including heteroaryl, -COR₇, SO₂R₇, -C(S) NHR₇, -C(NH)NHR₇, -COR₁₀, 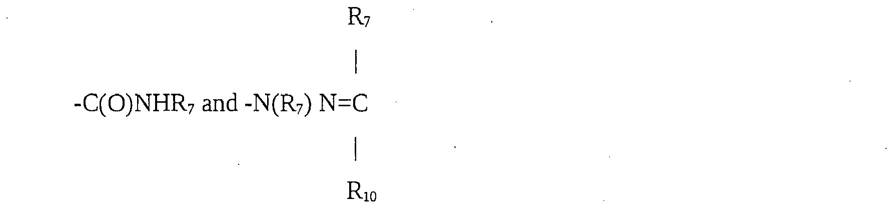 where R₇ is selected from the group consisting of H, alkyl and aryl including heteroaryl provided R₇ may be the same or different for R₁ and R₃ in the same compound;
R₂ is selected from the group consisting of F, Cl, Br, I, OR₇, NO₂, alkyl, aryl including heteroaryl, formyl, acyl, C(O)NR₇R₁₀, C(O)OR₇, NR₇R₁₀, N=C(R₇)(R₁₀), SR₇, SO₂NH₂, SO₂ alkyl and SO₂aryl,
and m is 0, 1 or 2;
R₃ is selected from the group consisting of R₇, OR₇, N(R₇) (R₁₀), N=C(R₇) (R₁₀), N(R₇) N(R₇) (R₁₀), N(R₇) N=C(R₇) (R₁₀) and CH(R₇)C(O)R₈;
where R₈ is selected from the group consisting of R₇, OR₇ and NR₇R₁₀;
R₉ is selected from the group consisting of hydrogen, alkyl, aryl including heteroaryl, C(O)R₁₀, -SO₂R₁₀, -C(S)NHR₁₀, -C(NH) NH (R₁₀) and -C(O) NHR₁₀,
R₁₀ is selected for the group consisting of H, alkyl or aryl including heteroaryl and in each case may be the same or different from substituent R₇, provided R₁₀ may be the same or different for R₁ and R₃ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, BF₄⁻ and PF₆⁻ or null;
with proviso that,
(i) when two alkyl groups are present on the same carbon or nitrogen, they may be linked together to form a cyclic structure ;
(ii) the nitrogen of heteroaryl ring of R₁₀, when present, may be quaternized;
(iii) when R₃ is OR₇ and R₁ is -NHNH₂ then R₇ is not alkyl and
(iv) when R₃ is OR₇, R₁ is N(R₇)(NR₇)R₉ and R₉ is C(O)R₁₀ where
R₁₀ is alkyl, then R₇ is not hydrogen.
and/or a compound represented by formula (II) or its pharmaceutically acceptable salt thereof, 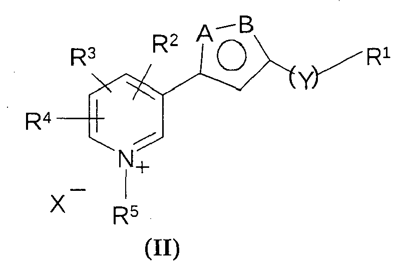 wherein,
R¹ is alkyl or aryl group;
Y is selected from the group comprising of sulfur, oxygen, nitrogen, or alkylene;
A and B are independently selected from nitrogen, NH, NR6, sulfur, oxygen or carbon to form heteroaromatic ring system;
R², R³ and R⁴ are independently selected from the group consisting of F, Cl, Br, I, OR⁷, NO₂, alkyl, aryl including heteroaryl, formyl, acyl, C(O)NR⁶R⁷, C(O)OR⁶, NR⁶R⁷, N=C(R⁶)(R⁷), SR⁶, SO₂NH₂, SO₂alkyl, SO₂aryl; R², R³ and R⁴ might be optionally joined together to form a ring system;
R⁵ is independently selected for the group consisting of alkyl, aryl and null;
R⁶ is independently selected from the group consisting of H, alkyl and aryl including heteroaryl provided R⁶ might be different for R², R³ and R⁴ in the same compound;
R⁷ is independently selected from the group consisting of H, alkyl and aryl including heteroaryl and in each case optionally different from substituent R⁶, provided R⁷ might be different for R², R³ and R⁴ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogenion; phosphonate ion, phosphate ion, BF4, PF6 and null;
with proviso that when two alkyl groups are present on the same carbon or nitrogen, they are optionally linked together to form a cyclic structure;
and at least one agent for treatment of heart failure or pharmaceutically acceptable salts thereof.

2. The pharmaceutical combination according to claim 1, wherein the compound of formula (I) or (II) is selected from:
1-(2-ethoxy -2- oxoethyl) -4-(phenylsulfonyl hydrazino carbonyl) pyridinium bromide (compound 1);
1-(2-phenylamino-2-oxoethyl)-4- (phenylsulfonyl hydrazino carbonyl) pyridinium chloride (compound 2);
1-(2-ethoxy -2- oxoethyl) -3-(phenylsulfonyl hydrazino carbonyl) pyridinium bromide (compound 3);
1-(2-(2',4'-dichlorophenyl)-2-oxoethyl)-3-(2(methoxy)ethyloxycarbonyl) pyridinium bromide (Compound 4);
1-(2-phenylamino-2-oxoethyl)-3-((benzoyloxy)ethylaminocarbonyl) pyridinium chloride (compound 5);
1-(2-thien-2'-yl- 2-oxoethyl)-3-(phenylaminocarbonyl hydrazinocarbonyl)pyridinium bromide (compound 6);
1-(2-phenyl-2-oxoethyl)-3-(2-(acetoxy)ethylaminocarbonyl) pyridinium bromide (compound 7);
1-(2-phenylamino-2-oxoethyl)-3-(phenyl sulfonyl hydrazino carbonyl) pyridinium chloride (compound 8);
1-(2-phenylamino-2-oxoethyl)-3-((4-methylphenyl) sulfonyl hydrazino carbonyl)pyridinium chloride (compound 9);
1-(2-phenyl-2-oxoethyl)-3-(2-(benzoyloxy)ethyloxy carbonyl) pyridinium bromide (compound 10);
1-(2-thien-2'-yl-2-oxoethyl)-3-(phenylcarbonyl hydrazino carbonyl) pyridinium bromide (compound 11);
1-(2-ethoxy-2-oxoethyl)-3-((phenylmethyl)sulfonyl hydrazino carbonyl)pyridinium bromide (compound 12);
1-(2-phenyl-2-oxoethyl)-3-((phenylmethyl)sulfonyl hydrazino carbonyl) pyridinium bromide (compound 13);
N, N' - bis [3-carbonyl-1-(2-furan-2'-yl-2-oxoethyl) pyridinium] hydrazine dibromide.
(Compound No: 14);
N, N'-bis [3-carbonyl-1- (2-thien-2'-yl-2-oxoethyl) pyridinium] hydrazine dichloride.
(Compound No: 15);
1-(2-thien-2'-yl-2-oxoethyl)-3-((2-(1-oxo-3-cyclohexyl)-propyl)-hydrazino carbonyl)-pyridinium bromide. (Compound No: 16);
1-(2-phenylamino-2-oxo ethyl)-3-({2-(1-oxo-3-cyclohexyl)-propyl}-hydrazino-carbonyl}-pyridinium bromide.(Compound No: 17);
1-(2-thien-2'-yl-2-oxoethyl)-3-[2-(benzoyloxy)ethylamino carbonyl]-pyridinium bromide (Compound No: 18);
1-(4-ethoxy-2, 4-dioxobutyl)-3-(2-(benzoyloxy)ethylamino carbonyl)-pyridinium chloride. (Compound No: 19);
1-(2', 4'-dichloro-phenyl-2-oxoethyl)-3-(2-methoxyethyl aminocarbonyl)-pyridinium bromide. (Compound No: 20);
N,N'-bis-[3-carbonyl-1-(2-cyclopropylamino-2-oxoethyl) pyridinium] hydrazine dichloride. (Compound No: 21);
1-(2-cyclopropylamino-2-oxoethyl)-3-(2-methoxyethylaminocarbonyl)-pyridinium chloride. (Compound No: 22);
N-N'-bis [3-carbonyl-1-(2-isopropylamino-2-oxoethyl) pyridinium] hydrazine dichloride. (Compound No: 23);
1-(2-thien-2'yl-2-oxoethyl)-3-(2-(2-chloro-3-pyridoylhydrazinocarbonyl) -pyridinium chloride. (Compound No: 24);
1-(2-isopropylamino-2-oxoethyl)-3-(2-methylsulfonylhydrazinocarbonyl) -pyridinium chloride. (Compound No: 25);
1-(2-(1-pyrrolidinyl)-2-oxoethyl)-3-(methanesulfonyl hydrazino carbonyl) pyridinium chloride.(Compound No: 26);
1-(2-thien-2'-yl-2-oxoethyl)-3-(methanesulfonyl hydrazino carbonyl) pyridinium chloride. (Compound No: 27);
N,N'-bis[3-carbonyl-1-(2-hydroxy-2-oxoethyl)pyridinium]hydrazine dichloride (Compound No: 28);
1-(2-thien-2'-yl-2-oxoethyl)-3-((2-methoxy ethyl) amino carbonyl)-5-bromo pyridinium chloride (Compound No: 29);
1-(2'-thien-2'-yl-2-oxoethyl)-3-[1-oxo-1-(2-methoxy carbonyl)pyridyl] hydrazino pyridinium chloride. (Compound No: 30);
1-[1-(2-thien-2'-yl-2-oxoethyl)-6-methyl-3-carbonyl pyridinium]-2-[1-(2-Thien-2'-yl-2-oxoethyl)-3-carbonyl pyridinium] hydrazine dichloride(compound no: 31);
1-(2-thien-2'-yl-2-oxoethyl)-3-(isopropylsulfonyl hydrazino carbonyl) pyridinium bromide(compound no: 32);
1-(2-(4-benzyl piperidin-1-yl)-2-oxoethyl)-3-(methanesulfonyl hydrazino carbonyl) pyridinium chloride(compound no: 33);
1-(2-(2-ethoxy carbonyl pyrrolidin-1-yl)-2-oxoethyl)-3-(methanesulfonyl hydrazino carbonyl) pyridinium chloride. (compound no: 34);
1-(2-thien-2'-yl-2-oxoethyl)-3-(methanesulfonyl hydrazino carbonyl)-5-bromo pyridinium bromide. (compound no: 35);
1-(2-thien-2'-yl-2-oxoethyl)-3-(ethoxycarbonyl hydrazino carbonyl) pyridinium bromide. (compound no: 36);
1-(2-(5-chloro-thien-2-yl)-2-oxoethyl)-3-(methanesulfonyl hydrazino carbonyl) pyridinium bromide (compound no: 37);
N,N'-bis[3-carbonyl-1-(2-(4-nitro-thien-2-yl)-2-oxoethyl)pyridinium] hydrazine dichloride. (compound no: 38);
1-(2-thien-2'-yl-2-oxoethyl)-3-(methanesulfonyl hydrazino carbonyl) -6-methyl pyridinium bromide. (compound no: 39);
N,N'-bis[3-carbonyl-1-(2-(5-methyl-thien-2-yl)-2-oxoethyl) pyridinium] hydrazine dichloride. (compound no: 40);
N,N'-bis[3-carbonyl-1-(2-(2-ethoxycarbonyl pyrrolidin-1-yl)-2-oxoethyl) pyridinium] hydrazine dichloride. (compound no: 41);
1-[1-(2-thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 42);
1-(2-(4-carbethoxy-thiazolidin-3-yl)-2oxoethyl)-3-(methanesulfonyl hydrazino carbonyl) pyridinium chloride (compound no: 43);
N,N'-bis[3-carbonyl-1-(2-(5-chloro-thien-2-yl)-2-oxoethyl) pyridinium] hydrazine dichloride. (compound no: 44);
1-(2-(5-methyl-thien-2-yl)-2-oxoethyl)-3-(methanesulfonyl hydrazino carbonyl) pyridinium chloride (compound no: 45);
1-(2-(4-nitro-thien-2-yl)-2-oxoethyl)-3-(methanesulfonyl hydrazino carbonyl) pyridinium bromide. (compound no: 46);
1-(2-phenylamino-2-oxoethyl)-3-(phenyl hydrazino carbonyl) pyridinium chloride (compound no: 47);
1-(2-phenylamino-2-oxoethyl)-4 -[2-(benzoyloxy) ethylamino carbonyl] pyridinium chloride (compound no: 48);
1-2-(5-nitro-thien-2-yl)-2-oxoethyl)-3-(methanesulfonyl hydrazino carbonyl) pyridinium chloride (compound no: 49);
1-(2-thien-2'-yl-2-oxoethyl)-3-(trifluoromethanesulfonyl hydrazino carbonyl) - pyridinium bromide. (compound no. 50);
1-(2-thien-2'-yl-2-oxoethyl)-3-(phenyl hydrazino carbonyl) pyridinium bromide (compound no.51);
1-(2-thien-2'-yl-2-oxoethyl)-3-(p-methoxy phenyl sulfonyl hydrazino carbonyl) pyridinium bromide (compound no. 52);
1-(2-ethoxy-2-oxoethyl)-3-(phenyl aminocarbonyl hydrazino carbonyl) pyridinium bromide (compound no. 53);
1-(2-ethoxy-2-oxoethyl)-3-(p-toluene sulfonyl hydrazino carbonyl) pyridinium bromide (compound no. 54);
1-(2-phenyl-2-oxoethyl)-3-(phenylamino carbonyl hydrazino carbonyl) pyridinium bromide (compound no. 55);
1-(2-phenylamino-2-oxoethyl)-3-(benzyl sulfonyl hydrazino carbonyl) pyridiniumchloride. (compound no. 56);
1-(2-phenyl-2-oxoethyl)-4-(methanesulfonyl hydrazino carbonyl) pyridinium bromide (compound no. 57);
1-(2-phenyl-2-oxoethyl)-3-(phenyl hydrazino carbonyl) pyridinium bromide. (compound no. 58);
1-(2-ethoxy-2-oxoethyl)-4-[2-(benzoyloxy) ethyl amino carbonyl] pyridinium bromide (compound no. 59);
1-(2-ethoxy-2-oxoethyl)-3-(phenyl hydrazino carbonyl) pyridinium bromide. (compound no. 60);
1-(2-phenyl-2-oxoethyl)-3-(p-methoxyphenyl sulfonyl hydrazino carbonyl) pyridinium bromide. (compound no. 61);
1-(2-phenyl-2-oxoethyl)- 4-[2-(benzoyloxy) ethyl amino carbonyl] pyridinium bromide. (compound no. 62);
1-(2-ethoxy-2-oxoethyl)- 4-(p-methanesulfonyl hydrazino carbonyl) pyridinium bromide. (compound no. 63);
N, N'-Bis [3-carbonyl-1- (2-phenyl-2-oxoethyl)-pyridinium] hydrazine dibromide (compound 64);
N, N'-Bis [3-carbonyl-1-(2-ethoxy-2-oxoethyl) pyridinium] hydrazine dibromide (compound 65);
N, N'-Bis [3-carbonyl-1- (2- (2, 4-dichlorophenyl)-2-oxoethyl) pyridinium] hydrazine dibromide (compound 66);
1- (2-Ethoxy-2-oxoethyl)-3- (2- (2-pyridyl) hydrazinocarbonyl) pyridinium bromide (compound 67);
1- (2-Thien-2'-yl-2-oxoethyl)-3- (methanesulfonyl hydrazinocarbonyl) pyridinium bromide (compound 68);
N, N'-Bis [3-carbonyl-1- (2-thien-2'-yl-2-oxoethyl) pyridinium] hydrazine dibromide (compound 69);
1- (2-Ethoxy-2-oxoethyl)-3- (2- (benzoyloxy) ethylaminocarbonyl) pyridinium bromide (compound 70);
1- (2- (2,4-Dichlorophenyl)-2-oxoethyl)-3- (2-(benzoyloxy) ethylamino- carbonyl) pyridinium bromide (compound 71);
1- (2-Thien-2'-yl-2-oxoethyl)-3- (2- (2-pyridyl) hydrazinocarbonyl) pyridinium bromide (compound 72);
1- (2-Phenyl-2-oxoethyl)-3- (2- (2-pyridyl) hydrazinocarbonyl) pyridinium bromide (compound 73);
1- (2-Phenyl-2-oxoethyl)-3- (hydrazinocarbonyl) pyridinium bromide (compound 74);
1- (2- Phenyl-2-oxoethyl)-3- (methanesulfonyl hydrazinocarbonyl) pyridinium bromide (compound 75);
1- (2-Ethoxy-2-oxoethyl)-3- (methanesulfonyl hydrazinocarbonyl) pyridinium bromide (compound 76);
1- (2-Phenyl-2-oxoethyl)-3- (phenylsulfonylhydrazino carbonyl) pyridinium bromide (compound 77);
1- (2-Phenyl-2-oxoethyl)-2-chloro-3- (phenylsulfonylhydrazino carbonyl) pyridinium bromide (compound 78);
1- (2- Phenyl-2-oxoethyl)-3- (2- (methoxy) carbonyl) ethyloxy carbonyl pyridinium bromide (compound 79);
1- (2-Ethoxy-2-oxoethyl)-3- (2- (benzoyloxy) ethyloxy carbonyl) pyridinium bromide (compound 80);
1-(2-Thien-2'-yl-2-oxoethyl)-4-(2-(benzoyloxy) ethylaminocarbonyl) pyridinium bromide (compound 81);
1- (2-thien-2'-yl-2-oxoethyl)-3- [(3-phenyl methyl)pyrazol-5-yl] pyridinium bromide (compound 82);
1-(2-thien-2'-yl-2-oxoethyl)-3-[(3-phenyl methyl) oxazol-5-yl pyridinium bromide (compound 83);
1-(2-thien-2'-yl-2-oxoethyl)-3-[3-{1-(2-thien-2'-yl)-2-oxoethyl pyridinium-4 thio} methyl-pyrazol-5-yl] pyridinium dibromide. (compound 84);
1- (2-thien-2'-yl-2-oxoethyl)-3- [3- f 1- (3, 5-dimethylpyrazol-1-yl) methyl} pyrazol-5-yl] pyridinium bromide. (compound 85);
1- (2-thien-2'-yl-2-oxoethyl)-3- [3-phenylmethyl}-1- {2-pyridyl}-pyrazol-5-yl]-pyridinium bromide. (compound 86);
1- (2-thien-2'-yl-2-oxoethyl)-3- [3 (3, 5-dimethylpyrazol-1-yl) methyl-1-pyridyl} pyrazol-5-yl] pyridinium bromide. (compound 87);
1- [2- (cyclopropylamino)- 2-oxoethyl] 3- [3- (3, 5-dimethylpyrazol-1-yl) methyl}-pyrazol-5-yl]-pyridinium bromide. (compound 88);
1- {2-(4-nitro-2-thienyl)-2-oxoethyl}-3-[3{(3,5-dimethylpyrazol-1-yl) methyl} pyrazol-5-yl]-pyridinium bromide. (compound 89);
1- (2-cyclopropylamino-2-oxoethyl)-3 [(3-phenylmethyl) pyrazol-5-yl] pyridinium chloride. (compound 90);
3,5-bis-[1-(2-thien-2'-yl-2-oxoethyl)-pyridinium-3-yl]-pyrazole dibromide. (compound 91);
1-(2-thien-2'-yl-2-oxoethyl)-3-1 (l-phenyl-3-phenylmethyl) pyrazol-5-yl] pyridinium chloride. (compound 92);
1-(2-(5'-methyl-2-thienyl)-2-oxoethyl)-3-[(3-phenylmethyl) pyrazol-5-yl] pyridinium chloride. (compound 93);
1- (2-thien-2'-yl-2-oxoethyl) 3- [1-phenyl, 3- { (3, 5-dimethyl pyrazol-1-yl) methyl)} pyrazol-5-yl]-pyridinium chloride. (compound 94);
1- (2-phenyl-2-oxoethyl)-3- [(3-phenylmethyl) pyrazol-5-yl]-pyridinium bromide. (compound 95);
1- (2-cyclopropylamino- 2-oxoethyl) 3- [(1-phenyl-3-phenylmethyl) pyrazol-5 yl]-pyridinium chloride (compound 96);
1- (2- (4-benzyl-l-piperidinyl)-2-oxoethyl) 3- [(3-phenoxymethyl) pyrazol-5-yl] pyridinium bromide (compound 97);
1 (2-phenyl-2-oxoethyl)-3-[(3-(3, 5-dimethylpyrazol-1-yl) methyl) pyrazol-5 yl]-pyridinium chloride (compound 98);
1-(2-(5-methyl-2-thienyl)-2-oxoethyl)-3-[(3,-(3, 5-dimethylpyrazol-1-yl) methyl). pyrazol-5-yl] pyridinium chloride (compound 99);
1- (2-phenyl-2-oxoethyl)-3- [(1-phenyl-3-phenylmethyl) pyrazol-5-yl] pyridinium chloride (compound 100);
1- (2- (5-methyl-2-thienyl)-2-oxoethyl)-3- [(3 (2-cyclohexyl ethyl) pyrazol-5-yl] pyridinium chloride (compound 101);
1- (2-cyclopropylamino-2-oxoethyl)-3- [(3- (2-cyclohexylethyl) pyrazol-5-yl] pyridinium chloride (compound 102);
1- (2-phenyl-2-oxoethyl)-3- [(3- (2-cyclohexylethyl) pyrazol-5-yl] pyridinium chloride (compound 103);
1- (2-cyclopropylamino-2-oxoethyl)-3- [(1-cyclohexyl-3-phenylmethyl) pyrazol 5-yl] pyridinium chloride (compound 104);
1- (2-thien-2'-yl-2-oxoethyl)-3- [(3-phenoxymethyl) pyrazol-5-yl] pyridinium chloride (compound 105);
1- [2- (l-adamantylamino)-2-oxoethyl]-3- [(3-phenylmethyl) pyrazol-5-yl] pyridinium chloride (compound 106);
1- (2-phenyl-2-oxoethyl)-3- [{3- (3, 5-dimethylpyrazol-1-yl) methyl)} 1-phenyl-pyrazol-5-yl] pyridinium bromide (compound 107);
1-(2-(4-nitor-2-thienyl)-2-oxoethyl)-3-[(1-cyclohexyl-3-(3,5-dimethylpyrazol-1 yl)-methyl) pyrazol-5-yl] pyridinium bromide (compound 108);
1- (2- (4-nitro-2-thienyl)-2-oxoethyl)-3 [(3- (2-cyclohexylethyl) pyrazol-5-yl] pyridinium bromide (compound 109);
1-(2-thien-2'-yl-2-oxoethyl)-3-[(1-phenyl-3-phenoxymethyl) pyrazol-5-yl] pyridinium chloride (compound 110);
1- (2- (4-nitro-2-thienyl)-2-oxoethyl)-3- [(1-phenyl-3-phenylmethyl) pyrazol-5 yl] pyridinium bromide (compound 111);
pyrazolel- (2-cyclopropylamino-2-oxoethyl)-3- [(3-phenoxymethyl) pyrazol-5-yl] pyridinium chloride (compound 112);
1- (2-cyclopropylamino-2-oxoethyl)-3- [(1-cyclohexyl-3- (3, 5-dimethyl pyrazole) - 1-yl)-5-yl]pyridinium chloride (compound 113);
1- (2- (5-chloro-2-thienyl)-2-oxoethyl)-3- [(3-phenoxymethyl) pyrazol-5-yl] pyridinium bromide (compound 114);
1-(2-phenyl-2-oxoethyl)-3-[(1-phenyl-3-phenoxyrnethyl) pyrazol-5-yl] pyridinium chloride (compound 115);
1- (2-thien-2'-yl-2-oxoethyl)-3- [(1-cyclohexyl-3- (3, 5-dimethylpyrazol-1-yl-methyl) pyrazol-5-yl] pyridinium chloride (compound 116);
1- (2-cyclopropylamino-2-oxoethyl)-3- [(1-phenyl-3-phenoxymethyl) pyrazol-5 yl] pyridinium bromide (compound 117);
1- (2-thien-2'-yl-2-oxoethyl)-3- [(1-phenyl-3- (2-cyclohexylethyl)pyrazol-5-yl] pyridinium bromide (compound 118);
1-(2-thien-2'-yl-2-oxoethyl)-3-[(1-cyclohexyl-3-phenoxymethyl)pyrazol-5-yl] pyridinium chloride (compound 119);
3- [(3-phenylmethyl)pyrazol-5-yl] pyridine hydrochloride (compound 120);
3- [(3-phenoxymethyl) pyrazol-5-yl] pyridine hydrochloride (compound 121);
3- [(3, 5-dimethylpyrazol-1-yl-methyl) pyrazol-5-yl] pyridine (compound 122);
3- [3- (2-cyclohexyl-ethyl)-pyrazol-5-yl] pyridine (compound 123);
1- (2-napthyl-2-oxo ethyl)-3 [(3-phenoxymethyl) pyrazol-5-yl] pyridinium bromide (compound 124);
1- (phenylmethyl)-3 [(3-phenyl methyl)pyrazol-5-yl] pyridinium chloride (compound 125);
1-(2-thien-2'-yl-2-oxo ethyl)-3[(3 (-1-naphthyl) pyrazol-5-yl] pyridinium chloride (compound 126);
1- (2-phenyl-2oxoethyl)-3 [3 (thienyl-2-yl-methyl)pyrazol-5-yl] pyridinium chloride (compound 127);
1- (2- (5-methyl-2-thienyl)-2-oxoethyl)-3- [3 (2-phenyl ethyl) pyrazol-5 yl] pyridinium chloride (compound 128);
1- (2- (5-methyl 2-thienyl)-2-oxo ethyl)-3- [3- (3-phenoxy propyl) pyrazol-5 yl] pyridinium chloride (compound 129);
1- (isopropyl)-3 [(3-phenylmethyl) pyrazol-5-yl] pyridinium bromide (compound 130);
1- (2- (5-methyl-2-thienyl)-2-oxoethyl)-3- [(3-thiophenylmethyl) pyrazol-5 yl] pyridinium chloride (compound 131);
1- (2-thien-2'-yl-2-oxoethyl)-3 [(3- (N-methyl-indole-3-yl methyl) pyrazol-5 yl] pyridinium chloride (compound 132);
1- (2-napthyl-2-oxo-ethyl)-3 [(3-methyl) pyrazol-5-yl] pyridinium bromide (compound 133);
1- (2- (1, 4 benzodioxane-6-yl-amino-2-oxoethyl)-3 [(3-phenylmethyl) pyrazol-5-yl] pyridinium chloride (compound 134);
1- (2-thien-2'-yl-2-oxo. ethyl)-3[(3-phenyl) pyrazol-5-yl]-5 bromo pyridinium chloride (compound 135);
1- (2-thien-2'-yl)-2-oxoethyl)-3 [(3-phenyl) pyrazol-5-yl] quinolinium chloride (compound 136) and
3- [(3-phenyl) pyrazol-5-yl)] quinoline (compound 137).

3. The pharmaceutical combination according to claim 1, wherein compound of formula (I) is 1-(2-thien-2'-yl-2-oxoethyl)-3-(methanesulfonyl hydrazino carbonyl) pyridinium chloride.

4. The pharmaceutical combination according to claim 1, wherein an agent for treatment of heart failure is selected from digitalis glycoside and phosphodiesterase inhibitor including pharmaceutically acceptable salts thereof.

5. The pharmaceutical combination according to claim 4, wherein digitalis glycoside is digitoxin, gitoxin, gitalin, digoxin, F-gitonin, digitonin, or pharmaceutically acceptable salts thereof; and/or wherein phosphodiesterase inhibitor is amrinone, milrinone, enoximone, bucladesine or pharmaceutically acceptable salts thereof.

6. The pharmaceutical combination according to any of the preceding claims comprising 1-(2-thien-2'-yl-2-oxoethyl)-3-(methanesulfonyl hydrazino carbonyl) pyridinium chloride and at least one agent selected from digitoxin, gitoxin, gitalin, digoxin, F-gitonin, digitonin, amrinone, milrinone, enoximone, bucladesine or pharmaceutically acceptable salts thereof.

7. A pharmaceutical combination according to claim 1 for use in a method for treatment or prevention of diabetes and aging-related vascular complications, selected from diabetic cardiomyopathy, hypertensive cardiomyopathy, and dilated cardiomyopathy.

8. A pharmaceutical combination according to claim 1 for use in a method for treatment or prevention of the disease condition selected from the group consisting of congestive heart failure of diverse etiology like left ventricular dysfunction (systolic and diastolic), ischemic cardiomyopathy, hypertrophic cardiomyopathy, diabetic cardiomyopathy, hypertensive cardiomyopathy, dilated cardiomyopathy and metabolic cardiomyopathy (thyroid, carcinoid, pheochromocytoma or acromegaly associated) and myocardial infarction and its sequelae.

9. A pharmaceutical combination according to claim 1 for use in a method for the treatment of the disease condition related to diabetes or aging-related vascular complications, preferably congestive heart failure and more preferably diabetes associated congestive heart failure.

10. Use of compound of formula (I) and/or (II) as defined in claim 1 or pharmaceutically acceptable salts thereof in association with pharmaceutically acceptable carrier for the preparation of medicament for treatment of heart failure.
